(19) Europäisches Patentamt
European Patent Office
Office européen des brevets

(11) EP 2 874 597 B1

# (12) EUROPEAN PATENT SPECIFICATION

(45) Date of publication and mention of the grant of the patent:
**02.01.2019 Bulletin 2019/01**

(21) Application number: **13736924.5**

(22) Date of filing: **16.07.2013**

(51) Int Cl.:
*A61K 8/36* $^{(2006.01)}$ *A61K 8/37* $^{(2006.01)}$
*A61Q 1/10* $^{(2006.01)}$ *A61K 8/86* $^{(2006.01)}$
*A61K 8/92* $^{(2006.01)}$ *A61K 8/06* $^{(2006.01)}$
*A61K 8/81* $^{(2006.01)}$ *A61K 8/88* $^{(2006.01)}$

(86) International application number:
**PCT/EP2013/064978**

(87) International publication number:
**WO 2014/012918 (23.01.2014 Gazette 2014/04)**

## (54) COSMETIC COMPOSITION FOR COATING KERATINOUS FIBRES

KOSMETISCHE ZUSAMMENSETZUNG ZUR BESCHICHTUNG VON KERATINFASERN

COMPOSITION COSMÉTIQUE SERVANT À RECOUVRIR DES FIBRES DE KÉRATINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.07.2012 FR 1257061**
**15.10.2012 US 201261713744 P**

(43) Date of publication of application:
**27.05.2015 Bulletin 2015/22**

(73) Proprietor: **L'Oréal**
**75008 Paris (FR)**

(72) Inventors:
- **BARBA, Claudia**
  **F-75010 Paris (FR)**
- **LE MERRER, Carole**
  **F-92120 Montrouge (FR)**

(74) Representative: **Goudet, Sylvain**
**L'Oréal**
**Service DIPI**
**9 Rue Pierre Dreyfus**
**92110 Clichy (FR)**

(56) References cited:
**WO-A2-00/74519**
**WO-A2-2010/002602**
**FR-A1- 2 932 070**
**US-A- 4 822 602**
**US-A- 5 389 363**
**US-A1- 2004 013 625**
**US-A1- 2004 180 020**

- **"Glyceryl stearate ED" In: Michael and Irene Ash: "HANDBOOK OF INDUSTRIAL SURFACTANTS", 1 January 2000 (2000-01-01), SYNAPSE INFORMATION RESOURCES, INC, XP002696393, ISBN: 1-890595-21-7 pages 1152-1153,**
- **"Steareth-10 ED" In: Michael and Irene Ash: "HANDBOOK OF INDUSTRIAL SURFACTANTS", 1 January 2000 (2000-01-01), SYNAPSE INFORMATION RESOURCES, INC, XP002696394, ISBN: 1-890595-21-7 page 1403,**
- **"PEG-40 stearate ED" In: Michael and Irene Ash: "HANDBOOK OF INDUSTRIAL SURFACTANTS", 1 January 2000 (2000-01-01), SYNAPSE INFORMATION RESOURCES, INC, XP002696395, ISBN: 1-890595-21-7 page 1299,**
- **"Sorbitan tristearate ED" In: Michael and Irene Ash: "HANDBOOK OF INDUSTRIAL SURFACTANTS", 1 January 2000 (2000-01-01), SYNAPSE INFORMATION RESOURCES, INC, XP002696396, ISBN: 1-890595-21-7 page 1397,**
- **Anonymous: "Product guide", Global Seven INTERNET CITATION, 1 March 2009 (2009-03-01), pages 2FP, 1-31, XP002696397, Retrieved from the Internet: URL:http://www.globalseven.com/brochure.pd f [retrieved on 2013-04-30]**
- **"Lipocol O-3 ED" In: Michael and Irene Ash: "HANDBOOK OF INDUSTRIAL SURFACTANTS,", 1 January 2000 (2000-01-01), SYNAPSE INFORMATION RESOURCES, INC, XP002696398, ISBN: 1-890595-21-7 page 46,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 874 597 B1

- "APG 325 CS ED" In: Michael and Irene Ash: "HANDBOOK OF INDUSTRIAL SURFACTANTS", 1 January 2000 (2000-01-01), SYNAPSE INFORMATION RESOURCES, INC, XP002696399, ISBN: 1-890595-21-7 page 80,
- Anonymous: "Flexbond 381 Emulsion Polymer For Architectural Coatings", Ashland Inc. INTERNET CITATION, January 2009 (2009-01), XP002726635, Retrieved from the Internet: URL:http://perrychem.com/files/Flexbond381 .pdf [retrieved on 2014-07-03]
- "Stearyl alcohol" In: Tara E Gottschalck, Halyna P Breslawec: "International Cosmetic Ingredient Dictionary and Handbook", 2012, Personal Care Products Council vol. 2, pages 3204-3205,
- "Polyethylene" In: Tara E Gottschalck, Halyna P Breslawec: "International Cosmetic Ingredient Dictionary and Handbook", 2012, Personal Care Products Council vol. 2, pages 2492-2494,
- Anonymous: "Ditrimethylolpropane tetrastearate - Surfactants - SAAPedia - Page1", , 20 October 2014 (2014-10-20), XP055372953, Retrieved from the Internet: URL:http://www.saapedia.org/en/saa/?type=detail&id=5920 [retrieved on 2017-05-15]

## Description

The present invention relates to a cosmetic composition for coating keratinous fibres, in particular the eyelashes or eyebrows. In particular, the said cosmetic composition is a composition for making up and optionally caring for the eyelashes. The present invention also relates to a method for coating keratinous fibres, in particular to a method for making up and optionally caring for the eyelashes.

The composition used can in particular be provided in the form of a product for the eyelashes, such as a mascara, or a product for the eyebrows. More preferentially, the invention relates to a mascara. The term "mascara" is understood to mean a composition intended to be applied to the eyelashes: it can be a composition for making up the eyelashes, a base for making up the eyelashes (also known as base coat), a composition to be applied over a mascara, also known as top coat, or a composition for the cosmetic treatment of the eyelashes. The mascara is more particularly intended for human eyelashes but also for false eyelashes.

Mascaras are prepared in particular according to two types of formulation: water-based mascaras, known as cream mascaras, in the form of a dispersion of waxes in water; anhydrous mascaras or mascaras with a low water content, known as waterproof mascaras, in the form of dispersions of waxes in organic solvents. The present patent application relates more specifically to "water-based" mascaras.

Compositions for coating keratinous fibres with such a type of mascara generally consist of at least one fatty phase generally formed of one or more waxes dispersed in an aqueous liquid phase by means of an emulsifying system or conveyed in an organic solvent.

WO 2010/002602 A2 discloses mascara compositions that can be re-applied to build up the lash volume.

The application of mascara is targeted in particular at increasing the volume of the eyelashes and consequently at increasing the intensity of the gaze. Numerous thickening or volumizing mascaras exist to do this, the principle of which consists in depositing the maximum amount of material onto the eyelashes so as to obtain this volumizing (or loading) effect.

It is in particular through the amount of solid particles (especially the waxes, which make it possible to structure the composition) that the desired application specificities for the compositions can be adjusted, such as, for example, their fluidity or consistency, and also their thickening power (also known as the loading or make-up power).

These solid particles are dispersed in the cream mascara by means of an emulsifying system composed of one or more surface-active agent(s).

However, the make-up film obtained after applying these mascara compositions is generally not sufficiently resistant to water, for example during bathing or showering, to tears or to sweat, or even to sebum. The mascara then has a tendency to run - appearance of rings under the eyes - or to crumble over time: grains are deposited and unsightly marks appear around the eyes.

Furthermore, a mascara comprising many solid particles will tend to be difficult to spread over the eyelashes, which can cause uneven construction of the mascara deposit.

An aim of the present invention is to obtain a composition for coating keratinous fibres, preferably a mascara, which has good application properties in terms of slip and of playtime (redeposition, retouching).

An aim of the present invention is to obtain a composition for coating keratinous fibres, preferably a mascara, which has good lengthening properties for the eyelashes coated with such a composition.

An aim of the present invention is also to provide a composition for coating keratinous fibres which makes possible good separation of the eyelashes during its application, without formation of bundles of eyelashes, and while ensuring smooth and uniform deposition of material (without lumps of composition).

An aim of the present invention is thus to obtain a composition for coating keratinous fibres, preferably a mascara, which gives rise to a volumizing effect on the eyelashes.

An aim of the present invention is to obtain a composition for coating keratinous fibres, preferably a mascara, which has good staying power on the eyelashes.

An aim of the present invention is to obtain a composition for coating keratinous fibres, preferably a mascara, which has good curving properties for the eyelashes coated with such a composition.

An aim of the present invention is to obtain a composition for coating keratinous fibres, preferably a mascara, which has a good strength of black, from the point of view of colorimetry and chromaticity.

An aim of the present patent application is more particularly to provide a mascara in which not only the waxes but also the pigments are homogeneously dispersed, the said mascara exhibiting a sufficiently thick texture to obtain a loading deposit, of satisfactory consistency, which allows easy application to the eyelashes and a uniform deposition, that is to say one which is smooth and homogeneous.

In particular, an aim of the present invention is to produce a composition for coating keratinous fibres which shows good staying power and is highly resistant to rubbing actions and/or to water, in particular to ambient moisture, tears, sweat and/or sebum, but which is easily removed with warm water.

According to a first aspect, a subject-matter of the present invention is a cosmetic composition as defined in

claim 1. Such a ratio is also distinct from 0.

Surprisingly and unexpectedly, the inventors of the present patent application have solved this or these problem(s) by means of such a composition.

This composition thus gives rise to an emulsion-type mascara having a good cosmetic property, the material deposition of which takes place layer by layer in an unvarying and major way.

According to a second aspect, another subject-matter of the present invention is an assembly or kit for coating keratinous fibres, comprising:

- at least one cosmetic composition for coating keratinous fibres as described above, and
- at least one applicator for the composition, the said applicator comprising means, where appropriate as protruding elements, configured in order to engage with the said keratinous fibres, such as the eyelashes or eyebrows, so as to smooth and/or separate the eyelashes or eyebrows. Such protruding elements can comprise teeth, bristles or others. The said assembly, in particular the said applicator, can optionally be equipped with means for vibrating and/or heating the said composition.

According to a third aspect, another subject-matter of the present invention is an assembly or kit for packaging and applying a composition for coating keratinous fibres, comprising:

- a device for packaging the said cosmetic composition for coating keratinous fibres as described above,
- an applicator for the said composition.

The said applicator can be integral with a grasping member forming a cap for the said packaging device. In other words, the said applicator can be mounted in a removable position on the said device between a closed position and an open position of a dispensing aperture of the device for packaging the said composition.

According to a fourth aspect, another subject-matter of the present invention is a method for coating keratinous fibres, in particular for making up the eyelashes, comprising a stage of application of a cosmetic composition for coating keratinous fibres as described above.

The composition according to the invention advantageously comprises a solids content of greater than or equal to 35%, better still of greater than or equal to 38%, indeed even of greater than or equal to 40%, and advantageously of less than 55%.

Within the meaning of the present invention, the "*solids content*" denotes the content of non-volatile matter.

The solids content (abbreviated to SC) of a composition according to the invention is measured using a "Halogen Moisture Analyzer HR73" commercial halogen drying device from Mettler Toledo. The measurement is carried out on the basis of the weight loss of a sample dried by halogen heating and thus represents the percentage of residual matter once the water and the volatile matter have evaporated.

This technique is fully described in the documentation of the device supplied by Mettler Toledo.

The measurement protocol is as follows:

Approximately 2 g of the composition, hereinafter the sample, are spread out over a metal dish, which is introduced into the abovementioned halogen drying device. The sample is then subjected to a temperature of 105°C until an unchanging weight is obtained. The wet weight of the sample, corresponding to its initial weight, and the dry weight of the sample, corresponding to its weight after halogen heating, are measured using a precision balance.

The experimental error associated with the measurement is of the order of plus or minus 2%.

The solids content is calculated in the following way:

$$\text{Solids content (expressed as \% by weight)} = 100 \times (\text{dry weight/wet weight}).$$

The composition according to the invention advantageously comprises a rheology G* of less than 25 000 Pa, in particular of between 10 000 and 20 000 Pa. Such a measurement can be carried out with the Rheostress 600 of the Thermo Haake brand, via an oscillating cone-plate procedure. To do this, prepared mascara is placed on the thermostatically controlled plate using a spatula. The contact point is established with a gap of 0.3 mm by bringing down the cone which will be the contact with the mascara and the plate. The excess mascara is removed. The sample is regulated at 25°C for 180 seconds. G* is subsequently measured (at a constant temperature of 25°C) in a logarithmic scale from 1 Pa to 1.0 E+4 Pa at an amplitude of 1 Hz.

Throughout the description which follows and unless expressly mentioned:

- the term "alkyl" means a saturated, linear or branched, $C_8$-$C_{24}$, better still $C_{12}$-$C_{20}$ and more preferentially $C_{14}$-$C_{18}$ hydrocarbon chain.

- The term "acyl" means a saturated, linear or branched, $C_8$-$C_{24}$, better still $C_{12}$-$C_{20}$ and more preferentially $C_{14}$-$C_{18}$ hydrocarbon chain comprising a carboxyl functional group, the hydroxyl functional group (-OH) of which has been replaced.

According to specific preferred embodiments of the present invention:

- the said anionic surfactant(s) is (are) chosen from $C_{14}$-$C_{18}$ fatty acids, and preferably comprises stearic acid, more preferentially still is essentially composed of stearic acid, the stearic acid representing at least 75% by weight of the anionic surfactant(s) present in the composition;
- the said anionic surfactant(s) is (are) present at a total content of greater than or equal to 1% by weight, with respect to the total weight of the composition, preferably ranging from 1.5% to 8% by weight and better still from 2% to 5% by weight, with respect to the total weight of the composition;
- the said cationic counterion(s) is (are) chosen from a cation of inorganic origin, in particular chosen from alkali metal and alkaline earth metal cations, or of organic origin, preferably of organic origin;
- the said cationic counterion(s) is (are) chosen from ammonium, and its amine and aminoalcohol derivatives, or magnesium, preferably from ammonium, and its amine and aminoalcohol derivatives;
- the said cationic counterion(s) comprise(s) a primary (poly)hydroxyalkylamine, preferably aminomethyl propanediol;
- the total content of cationic counterion(s) is greater than or equal to 0.01% by weight, preferably inclusively between 0.1% and 5% by weight and better still between 0.5% and 4% by weight, with respect to the total weight of the composition;
- the non-ionic surfactant(s) exhibiting, at 25°C, an HLB balance within the Griffin meaning of greater than or equal to 8 are chosen from:

    - oxyethylenated and/or oxypropylenated glycerol ethers which can comprise from 1 to 150 oxyethylene and/or oxypropylene units;
    - oxyalkylenated, in particular oxyethylenated and/or oxypropylenated, alcohols which can comprise from 1 to 150 oxyethylene and/or oxypropylene units, preferably from 20 to 100 oxyethylene units;
    - esters of fatty acid, in particular $C_8$-$C_{24}$ and preferably $C_{16}$-$C_{22}$ fatty acid, and of polyethylene glycol (or PEG);
    - esters of fatty acid, in particular $C_8$-$C_{24}$ and preferably $C_{16}$-$C_{22}$ fatty acid, and of glycerol ethers which are oxyethylenated and/or oxypropylenated, preferably oxyethylenated;
    - esters of fatty acid, in particular $C_8$-$C_{24}$ and preferably $C_{16}$-$C_{22}$ fatty acid, and of sorbitol ethers which are oxyethylenated and/or oxypropylenated, preferably oxyethylenated;
    - dimethicone copolyol;
    - dimethicone copolyol benzoate;
    - copolymers of propylene oxide and of ethylene oxide, also known as EO/PO polycondensates;
    - and their mixtures;

- the non-ionic surfactant(s) exhibiting, at 25°C, an HLB balance within the Griffin meaning of greater than or equal to 8 is (are) present at a total content of greater than or equal to 1% by weight, with respect to the total weight of the composition, preferably ranging from 1.5% to 8% by weight and better still from 2% to 5% by weight, with respect to the total weight of the composition;
- the non-ionic surfactant(s) exhibiting, at 25°C, an HLB balance within the Griffin meaning of less than 8 are chosen from:

    - saccharide esters and ethers;
    - esters of fatty acids, in particular $C_8$-$C_{24}$ and preferably $C_{16}$-$C_{22}$ fatty acids, and of polyol, in particular of glycerol or sorbitol, preferably of glycerol;
    - oxyalkylenated, in particular oxyethylenated and/or oxypropylenated, alcohols;
    - a cyclomethicone/dimethicone copolyol mixture;
    - and their mixtures;

- the non-ionic surfactant(s) exhibiting, at 25°C, an HLB balance within the Griffin meaning of less than 8 is (are) present at a total content of greater than or equal to 1% by weight, with respect to the total weight of the composition, preferably ranging from 1.5% to 8% by weight and better still from 2% to 5% by weight, with respect to the total weight of the composition;
- the co-surfactant(s) is (are) chosen from fatty alcohols comprising from 12 to 24 carbon atoms and even better still from 14 to 22 carbon atoms;
- the emulsion can be of the wax(es)-in-water type;

- the said composition comprises a fatty phase dispersed in the aqueous phase;
- the fatty phase can predominantly comprise waxes;
- the said composition can comprise one or more wax(es), preferably several;
- this or these wax(es) can be chosen from beeswax, lanolin wax, Chinese insect waxes, rice wax, carnauba wax, candelilla wax, ouricury wax, esparto grass wax, cork fibre wax, sugarcane wax, Japan wax, sumach wax, montan wax, microcrystalline waxes, paraffin wax, ozokerite, polyethylene wax, the waxes obtained by the Fischer-Tropsch synthesis, a $C_{20}$-$C_{40}$ alkyl (hydroxystearyloxy)stearate, waxy copolymers, in particular the ethylene/vinyl acetate copolymer, and their esters, the waxes obtained by catalytic hydrogenation of animal or vegetable oils having linear or branched $C_8$-$C_{32}$ fatty chains, the waxes obtained by hydrogenation of olive oil esterified with stearyl alcohol, the waxes obtained by hydrogenation of castor oil esterified with cetyl alcohol, silicone waxes, such as alkyl or alkoxy dimethicones having from 16 to 45 carbon atoms, fluoro waxes, and their mixture(s);
- the said composition can comprise at least one wax chosen from candelilla wax, carnauba wax, beeswax, paraffin wax, polyethylene wax, rice wax, the wax resulting from an ethylene/vinyl acetate copolymer, and their mixture(s);
- the wax(es) can be present in a content of less than or equal to 12% by weight, with respect to the total weight of the composition, better still of less than or equal to 10% by weight, indeed even of less than or equal to 9% by weight, with respect to the total weight of the composition, in particular of between 5% and 8% by weight, with respect to the total weight of the composition;
- the organic lipophilic gelling agent(s) is (are) present at a content ranging from 0.5% to 6% by weight, on a dry basis, with respect to the total weight of the composition;
- the film-forming polymer(s) comprise(s) at least one aqueous dispersion of film-forming polymer, preferably at least two aqueous dispersions of film-forming polymer, advantageously at least one being an aqueous dispersion of acrylic polymer and at least the other being an aqueous dispersion of polyurethane, and their derivatives;
- the composition comprises at least one hydrophilic film-forming polymer in the soluble state in the aqueous phase and at least one hydrophilic gelling agent;
- the total solids content of film-forming polymer(s) is greater than or equal to 8% by weight, with respect to the total weight of the composition;
- the composition comprises at least one inorganic filler, advantageously chosen from talc, mica, synthetic fluorphlogopite, and their mixtures;
- the cosmetic composition can comprise at least one pulverulent colorant chosen from pigments, and preferably chosen from metal oxides, in particular iron oxides and titanium oxides;
- the metal oxide(s) is (are) preferably present at a content of greater than or equal to 2% by weight, with respect to the total weight of the composition, and advantageously inclusively between 3% and 15% by weight, with respect to the total weight of the composition;
- the composition can be a make-up composition, a make-up base, a "top coat" composition to be applied over a make-up, or a composition for the cosmetic treatment or care of keratinous fibres.

Other characteristics, properties and advantages of the present invention will become more clearly apparent on reading the description and examples which follow.

## Aqueous phase

The composition according to the invention comprises an aqueous phase, which can form a continuous phase of the composition.

The aqueous phase comprises water. It can also comprise at least one water-soluble solvent.

In the present invention, the term "water-soluble solvent" denotes a compound which is liquid at ambient temperature and which is miscible with water.

The water-soluble solvents which can be used in the compositions according to the invention can in addition be volatile.

Mention may in particular be made, among the water-soluble solvents which can be used in the compositions in accordance with the invention, of lower monoalcohols having from 1 to 5 carbon atoms, such as ethanol and isopropanol, and glycols having from 2 to 8 carbon atoms, such as ethylene glycol, propylene glycol, 1,3-butylene glycol and dipropylene glycol.

The aqueous phase (water and optionally the water-miscible solvent) is generally present in the composition according to the present patent application in a content ranging from 20% to 90% by weight, with respect to the total weight of the composition, preferably ranging from 25% to 80% by weight, preferentially ranging from 30% to 70% by weight and better still ranging from 35% to 60% by weight, with respect to the total weight of the composition.

**Fatty phase**

A fatty phase in accordance with the invention comprises at least one waxy phase.
This waxy phase comprises one or more wax(es).
This fatty phase can also comprise constituents chosen in particular from at least one pasty fatty substance, at least one volatile oil, at least one non-volatile oil, and their mixture(s).
The said fatty phase can predominantly comprise waxes.

Waxy phase or wax(es)

The wax(es) under consideration in the context of the present invention is (are) generally a lipophilic compound which is solid at ambient temperature (25°C), which has a reversible solid/liquid change in state and which has a melting point of greater than or equal to 30°C which can range up to 200°C and in particular up to 120°C.
In particular, the waxes which are suitable for the invention can exhibit a melting point of greater than or equal to 45°C and in particular of greater than or equal to 55°C.
Within the meaning of the invention, the melting point corresponds to the temperature of the most endothermic peak observed in thermal analysis (DSC) as described in Standard ISO 11357-3; 1999. The melting point of the wax can be measured using a differential scanning calorimeter (DSC), for example the calorimeter sold under the name DSC Q2000 by TA Instruments.
Preferably, the waxes exhibit an enthalpy of fusion $\Delta$Hf of greater than or equal to 70 J/g.
Preferably, the waxes comprise at least one crystallizable part, which is visible by X-ray diffraction observations.
The measurement protocol is as follows:
A sample of 5 mg of wax placed in a crucible is subjected to a first temperature rise passing from -20°C to 120°C at a heating rate of 10°C/minute, is then cooled from 120°C to -20°C at a cooling rate of 10°C/minute and is finally subjected to a second temperature rise passing from -20°C to 120°C at a heating rate of 5°C/minute. During the second temperature rise, the following parameters are measured:

- the melting point (M.p.) of the wax, as mentioned above corresponding to the temperature of the most endothermic peak of the melting curve observed, representing the variation of the difference in power absorbed as a function of the temperature,
- $\Delta$Hf: the enthalpy of fusion of the wax, corresponding to the integral of the entire melting curve obtained. This enthalpy of fusion of the wax is the amount of energy necessary to make the compound change from the solid state to the liquid state. It is expressed in J/g.

The wax(es) can be hydrocarbon wax(es), fluoro wax(es) and/or silicone wax(es) and can be of vegetable, mineral, animal and/or synthetic origin.
The wax(es) can be present at a content of less than or equal to 12% by weight, with respect to the total weight of the composition, better still of less than or equal to 10% by weight, more preferably of less than or equal to 9% by weight, with respect to the total weight of the composition.
According to a particularly preferred embodiment, a composition in accordance with the invention has a content of wax(es) ranging from 5% to 8% by weight, with respect to the total weight of the composition.
Use may preferably be made, as wax(es), of hydrocarbon waxes, such as beeswax, lanolin wax and Chinese insect waxes; rice wax, carnauba wax, candelilla wax, ouricury wax, esparto grass wax, cork fibre wax, sugarcane wax, Japan wax and sumach wax; montan wax, microcrystalline waxes, paraffin waxes and ozokerite; polyethylene waxes, the waxes obtained by the Fischer-Tropsch synthesis and waxy copolymers, in particular ethylene/vinyl acetate copolymers, and their esters.
Mention may also be made of waxes obtained by catalytic hydrogenation of animal or plant oils having linear or branched $C_8$-$C_{32}$ fatty chains.
Mention may in particular be made, among these, of hydrogenated jojoba oil, isomerized jojoba oil, such as the trans-isomerized partially hydrogenated jojoba oil manufactured or sold by Desert Whale under the commercial reference Iso-Jojoba-50®, hydrogenated sunflower oil, hydrogenated castor oil, hydrogenated coconut oil, hydrogenated lanolin oil, di(1,1,1-trimethylolpropane) tetrastearate, sold under the name Hest 2T-4S by Heterene, or di(1,1,1-trimethylolpropane) tetrabehenate, sold under the name Hest 2T-4B by Heterene.
Mention may also be made of silicone waxes, such as alkyl or alkoxy dimethicones having from 16 to 45 carbon atoms, or fluoro waxes.
Use may also be made of the wax obtained by hydrogenation of olive oil esterified with stearyl alcohol, sold under the name Phytowax Olive 18L57, or the waxes obtained by hydrogenation of castor oil esterified with cetyl alcohol, sold under the names Phytowax Ricin 16L64 and 22L73 by Sophim. Such waxes are described in Patent Application

FR-A-2 792 190.

The composition can comprise at least one non-polar wax. Preferably, the wax(es) comprise(s) one or more non-polar wax(es) chosen from polyethylene wax, paraffin wax, ozokerite, and their mixture(s).

The composition can comprise at least one polar wax. The term "polar wax" is understood to mean waxes comprising, in their chemical structure, in addition to the carbon and hydrogen atoms, at least one highly electronegative heteroatom, such as O, N or P.

Preferably, the wax(es) comprise(s) one or more polar wax(es) chosen from carnauba wax, candelilla wax, the wax resulting from an ethylene/vinyl acetate copolymer, natural (or bleached) beeswax and synthetic beeswax, and their mixture(s). Mention may be made, as synthetic beeswax, of the wax sold under the name Cyclochem 326 A by Evonik Goldschmidt (INCI name: Synthetic Beeswax).

The wax resulting from an ethylene/vinyl acetate copolymer can be a product sold under the name Coolbind by National Starch.

Preferably, the composition comprises a mixture of polar wax(es) and non-polar wax(es).

The composition can comprise at least one wax exhibiting a hardness ranging from 0.05 MPa to 15 MPa and preferably ranging from 6 MPa to 15 MPa. The hardness is determined by measuring the compressive force, measured at 20°C using the texture analyser sold under the name TA-TX2i by Rheo, equipped with a stainless-steel cylinder with a diameter of 2 mm, travelling at a measuring speed of 0.1 mm/s and penetrating the wax to a penetration depth of 0.3 mm.

According to a specific embodiment, the compositions in accordance with the invention can comprise at least one wax referred to as "tacky wax", that is to say having a tack of greater than or equal to 0.7 N.s and a hardness of less than or equal to 3.5 MPa.

The use of such a tacky wax can in particular make it possible to obtain a cosmetic composition which is easily applied to the eyelashes, which attaches well to the eyelashes and which results in the formation of a smooth, homogeneous and thickening make-up.

The tacky wax used can in particular have a tack ranging from 0.7 N.s to 30 N.s, in particular of greater than or equal to 1 N.s, especially ranging from 1 N.s to 20 N.s, in particular of greater than or equal to 2 N.s, especially ranging from 2 N.s to 10 N.s and in particular ranging from 2 N.s to 5 N.s.

The tack of the wax is determined by the measurement of the change in the force (compressive force or stretching force) as a function of the time at 20°C using the texture analyser sold under the name TA-TX2i® by Rheo, equipped with a spindle made of acrylic polymer in the shape of a cone forming an angle of 45°.

The measurement protocol is as follows:

The wax is melted at a temperature equal to the melting point of the wax + 10°C. The molten wax is cast in a receptacle with a diameter of 25 mm and a depth of 20 mm. The wax is recrystallized at ambient temperature (25°C) for 24 hours, so that the surface of the wax is flat and smooth, and then the wax is stored at 20°C for at least 1 hour before measuring the tack.

The spindle of the texture analyser is displaced at the rate of 0.5 mm/s and then penetrates the wax to a penetration depth of 2 mm. When the spindle has penetrated the wax to a depth of 2 mm, the spindle is held stationary for 1 second (corresponding to the relaxation time) and is then withdrawn at the rate of 0.5 mm/s.

During the relaxation time, the force (compressive force) strongly decreases until it becomes zero and then, during the withdrawal of the spindle, the force (stretching force) becomes negative to subsequently again increase towards the value 0. The tack corresponds to the integral of the curve of the force as a function of the time for the part of the curve corresponding to the negative values of the force (stretching force). The value of the tack is expressed in N.s.

The tacky wax which can be used generally has a hardness of less than or equal to 3.5 MPa, in particular ranging from 0.01 MPa to 3.5 MPa, especially ranging from 0.05 MPa to 3 MPa, indeed even ranging from 0.1 MPa to 2.5 MPa.

The hardness is measured according to the protocol described above.

Use may be made, as tacky wax, of a $C_{20}$-$C_{40}$ alkyl (hydroxystearyloxy)stearate (the alkyl group comprising from 20 to 40 carbon atoms), alone or as a mixture, in particular a $C_{20}$-$C_{40}$ alkyl 12-(12'- hydroxystearyloxy)stearate, of formula (II):

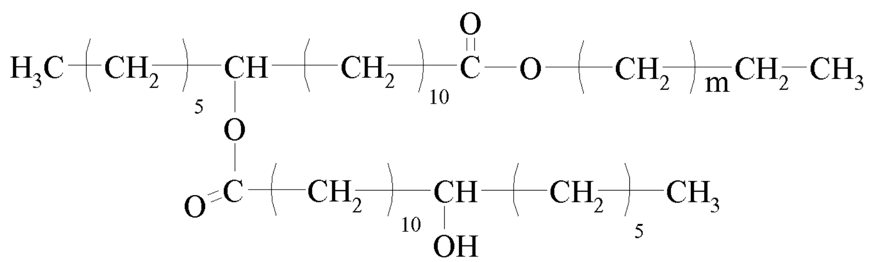

(II)

in which m is an integer ranging from 18 to 38, or a mixture of compounds of formula (II).

Such a wax is sold in particular under the names Kester Wax K 82 P® and Kester Wax K 80 P® by Koster Keunen.

The abovementioned waxes generally exhibit a starting melting point of less than 45°C.

Use may also be made of the microcrystalline wax sold under the reference SP18 by Strahl and Pitsch, which exhibits a hardness of approximately 0.46 MPa and a tack value of approximately 1 N.s.

The wax(es) can be present in the form of an aqueous wax microdispersion. The term "aqueous wax microdispersion" is understood to mean an aqueous dispersion of wax particles in which the size of the said wax particles is less than or equal to approximately 1 μm.

Wax microdispersions are stable dispersions of colloidal wax particles and are described in particular in "Microemulsions Theory and Practice", edited by L.M. Prince, Academic Press (1977), pages 21-32.

In particular, these wax microdispersions can be obtained by melting the wax in the presence of a surfactant and optionally of a portion of the water and then gradually adding hot water with stirring. The intermediate formation of an emulsion of the water-in-oil type, followed by phase inversion, with a microemulsion of the oil-in-water type finally being obtained, is observed. On cooling, a stable microdispersion of solid colloidal wax particles is obtained.

The wax microdispersions can also be obtained by stirring the mixture of wax, surfactant and water using stirring means, such as ultrasound, a high-pressure homogenizer or turbine mixers.

The particles of the wax microdispersion preferably have mean sizes of less than 1 μm (in particular ranging from 0.02 μm to 0.99 μm), preferably of less than 0.5 μm (in particular ranging from 0.06 μm to 0.5 μm).

The wax(es) and the surface-active agent(s) are present at a respective total content such that the ratio by weight of the wax(es) to the surface-active agent(s) is less than or equal to 1, better still less than or equal to 0.75, while being distinct from 0 and in particular between 0.2 and 0.6 inclusive.

<u>Pasty fatty substances</u>

A composition according to the invention can comprise at least one pasty fatty substance.

Within the meaning of the present invention, the term "pasty fatty substance" is understood to mean a lipophilic fatty compound exhibiting a reversible solid/liquid change in state and comprising, at a temperature of 23°C, a liquid fraction and a solid fraction.

In other words, the starting melting point of the pasty compound can be less than 23°C. The liquid fraction of the pasty compound, measured at 23°C, can represent from 9% to 97% by weight of the compound. This liquid fraction at 23°C preferably represents between 15% and 85% by weight and more preferably between 40% and 85% by weight.

Preferably, the pasty fatty substances exhibit an end melting point of less than 60°C.

Preferably, the pasty fatty substances exhibit a hardness of less than or equal to 6 MPa.

Preferably, the pasty fatty substances exhibit, in the solid state, a crystal organization which is visible by X-ray diffraction characterizations.

Within the meaning of the invention, the melting point corresponds to the temperature of the most endothermic peak observed in thermal analysis (DSC) as described in Standard ISO 11357-3; 1999. The melting point of a pasty substance or of a wax can be measured using a differential scanning calorimeter (DSC), for example the calorimeter sold under the name "DSC Q2000" by TA Instruments.

As regards the measurement of the melting point and the determination of the end melting point, the sample preparation and measurement protocols are as follows:
A sample of 5 mg of pasty fatty substance, preheated to 80°C and withdrawn with magnetic stirring using a spatula which is also heated, is placed in a hermetic aluminium capsule, or crucible. Two tests are carried out to ensure the reproducibility of the results.

The measurements are carried out on the abovementioned calorimeter. The oven is flushed with nitrogen. Cooling is carried out with an RCS 90 heat exchanger. The sample is subsequently subjected to the following protocol: it is first of all placed at a temperature of 20°C, is then subjected to a first temperature rise passing from 20°C to 80°C at a heating rate of 5°C/minute, is then cooled from 80°C to -80°C at a cooling rate of 5°C/minute and, finally, is subjected to a second temperature rise passing from -80°C to 80°C at a heating rate of 5°C/minute. During the second temperature rise, the variation in the difference in power absorbed by the empty crucible and by the crucible containing the sample of pasty substance or wax is measured as a function of the temperature. The melting point of the compound is the value of the temperature corresponding to the tip of the peak of the curve representing the variation in the difference in power absorbed as a function of the temperature.

The end melting point corresponds to the temperature at which 95% of the sample has melted.

The liquid fraction by weight of the pasty compound at 23°C is equal to the ratio of the enthalpy of fusion consumed at 23°C to the enthalpy of fusion of the pasty compound.

The enthalpy of fusion of the pasty compound is the enthalpy consumed by the compound in order to pass from the solid state to the liquid state. The pasty compound is said to be in the solid state when all of its mass is in the

crystalline solid form. The pasty compound is said to be in the liquid state when all of its mass is in the liquid form.

The enthalpy of fusion of the pasty compound is equal to the integral of the entire melting curve obtained using the abovementioned calorimeter, with a temperature rise of 5 or 10°C per minute, according to Standard ISO 11357-3:1999. The enthalpy of fusion of the pasty compound is the amount of energy necessary to make the compound change from the solid state to the liquid state. It is expressed in J/g.

The enthalpy of fusion consumed at 23°C is the amount of energy absorbed by the sample to change from the solid state to the state which it exhibits at 23°C, consisting of a liquid fraction and of a solid fraction.

The liquid fraction of the pasty compound measured at 32°C preferably represents from 30% to 100 % by weight of the compound, preferably from 50% to 100% by weight and more preferably from 60% to 100% by weight of the compound. When the liquid fraction of the pasty compound, measured at 32°C, is equal to 100%, the temperature of the end of the melting range of the pasty compound is less than or equal to 32°C.

The liquid fraction of the pasty compound, measured at 32°C, is equal to the ratio of the enthalpy of fusion consumed at 32°C to the enthalpy of fusion of the pasty compound. The enthalpy of fusion consumed at 32°C is calculated in the same way as the enthalpy of fusion consumed at 23°C.

As regards the measurement of the hardness, the sample preparation and measurement protocols are as follows:
The pasty fatty substance is placed in a mould with a diameter of 75 mm which is filled to approximately 75% of its height. In order to overcome the thermal history and to control the crystallization, the mould is placed in a Vötsch VC 0018 programmable oven, where it is first of all placed at a temperature of 80°C for 60 minutes, then cooled from 80°C to 0°C at a cooling rate of 5°C/minute, then left at the stabilized temperature of 0°C for 60 minutes, then subjected to a temperature rise passing from 0°C to 20°C at a heating rate of 5°C/minute and then left at the stabilized temperature of 20°C for 180 minutes.

The compressive force measurement is carried out with the TA/TX2i texture analyser from Swantech. The spindle used is chosen according to the texture:

- cylindrical steel spindle with a diameter of 2 mm, for very rigid starting materials;
- cylindrical steel spindle with a diameter of 12 mm, for relatively non-rigid starting materials.

The measurement comprises 3 stages: a first stage after automatic detection of the surface of the sample where the spindle is displaced at the measurement rate of 0.1 mm/s and penetrates the pasty fatty substance to a penetration depth of 0.3 mm - the software records the value of the maximum force achieved; a second "relaxation" stage where the spindle remains at this position for one second and where the force is recorded after 1 second of relaxation; finally, a third "withdrawal" stage where the spindle returns to its initial position at the rate of 1 mm/s and the probe withdrawal energy (negative force) is recorded.

The value of the hardness measured during the first stage corresponds to the maximum compressive force measured in newtons divided by the surface area of the cylinder of the texture analyser in contact with the pasty fatty substance, expressed in $mm^2$. The hardness value obtained is expressed in megapascals or MPa.

The pasty fatty substance is preferably chosen from synthetic compounds and compounds of vegetable origin. A pasty compound can be obtained by synthesis from starting materials of vegetable origin.

The pasty compound is advantageously chosen from:

- lanolin and its derivatives,
- petrolatum, in particular the product which has this as INCI name and which is sold under the name Ultima White PET USP by Penreco;
- polyol ethers chosen from polyalkylene glycol pentaerythrityl ethers, fatty alcohol ethers of sugars, and their mixtures, polyethylene glycol pentaerythrityl ether comprising five oxyethylene (5 OE) units (CTFA name: PEG-5 Pentaerythrityl Ether), polypropylene glycol pentaerythrityl ether comprising five oxypropylene (5 OP) units (CTFA name: PEG-5 Pentaerythrityl Ether), and their mixtures, and more especially the PEG-5 Pentaerythrityl Ether, PPG-5 Pentaerythrityl Ether and soybean oil mixture sold under the Lanolide name by Vevy, which is a mixture in which the constituents are in a 46/46/8 ratio by weight: 46% PEG-5 Pentaerythrityl Ether, 46% PPG-5 Pentaerythrityl Ether and 8% soybean oil;
- polymeric or non-polymeric silicone compounds,
- polymeric or non-polymeric fluorinated compounds,
- vinyl polymers, in particular:
    - olefin homopolymers and copolymers,
    - hydrogenated diene homopolymers and copolymers,
    - linear or branched oligomers, homo- or copolymers of alkyl (meth)acrylates preferably having a $C_8$-$C_{30}$ alkyl

group,

- oligomers, homo- and copolymers of vinyl esters having $C_8$-$C_{30}$ alkyl groups,
- oligomers, homo- and copolymers of vinyl ethers having $C_8$-$C_{30}$ alkyl groups,

- fat-soluble polyethers resulting from the polyetherification between one or more $C_2$-$C_{100}$ and preferably $C_2$-$C_{50}$ diols,
- esters,
- and/or their mixtures.

The pasty compound is preferably a polymer, in particular a hydrocarbon polymer.

Preference is given in particular, among the fat-soluble polyethers, to copolymers of ethylene oxide and/or of propylene oxide with long-chain $C_6$-$C_{30}$ alkylene oxides, more preferably such that the ratio by weight of the ethylene oxide and/or of the propylene oxide to the alkylene oxides in the copolymer is from 5:95 to 70:30. In this family, mention will be made in particular of copolymers such that the long-chain alkylene oxides are positioned in blocks having an average molecular weight from 1000 to 10 000, for example a polyoxyethylene/polydodecyl glycol block copolymer, such as the ethers of dodecanediol (22 mol) and of polyethylene glycol (45 OE) sold under the brand name Elfacos ST9 by Akzo Nobel.

Preference is given in particular, among the esters, to:

- esters of an oligomeric glycerol, in particular diglycerol esters,especially condensates of adipic acid and of glycerol, for which a portion of the hydroxyl groups of the glycerols have reacted with a mixture of fatty acids, such as stearic acid, capric acid, stearic acid and isostearic acid, and 12-hydroxystearic acid, preferably such as bis-diglyceryl polyacyladipate-2, sold under the brand name Softisan 649 by Sasol,
- arachidyl propionate, sold under the brand name Waxenol 801 by Alzo,
- phytosterol esters,
- fatty acid triglycerides and their derivatives, such as, for example, triglycerides of fatty acids, in particular $C_{10}$-$C_{18}$ fatty acids, which are partially or completely hydrogenated, such as those sold under the reference Softisan 100 by Sasol,
- pentaerythritolesters,

and their mixtures,

- esters of dimer diol and dimer diacid, if appropriate esterified on their free alcohol or acid functional group(s) by acid or alcohol radicals, in particular dimer dilinoleate esters; such esters can be chosen in particular from esters with the following INCI nomenclature: bis-behenyl/isostearyl/phytosteryl dimer dilinoleyl dimer dilinoleate (Plandool G), phytosteryl/isostearyl/cetyl/stearyl/behenyl dimer dilinoleate (Plandool H or Plandool S), and their mixtures,
- mango butter, such as that sold under the reference Lipex 203 by AarhusKarlshamn,
- shea butter, in particular that having the INCI name Butyrospermum Parkii Butter, such as that sold under the reference Sheasoft® by AarhusKarlshamn,
- and their mixtures,

The choice will preferably be made, among the pasty compounds, of bis-behenyl/isostearyl/phytosteryl dimer dilinoleyl dimer dilinoleate, bis-diglyceryl polyacyladipate-2, hydrogenated castor oil dimer dilinoleate, for example Risocast DA-L sold by Kokyu Alcohol Kogyo, hydrogenated castor oil isostearate, for example Salacos HCIS (V-L) sold by Nisshin Oil, mango butter, shea butter, vinylpyrrolidone/eicosene copolymers, or their mixture(s).

A composition according to the invention is preferably devoid of pasty fatty substance. However, a composition according to the invention can comprise one or more pasty fatty substances at a total content of greater than or equal to 0.01% by weight, with respect to the total weight of the composition, for example of between 0.1% and 2% by weight, with respect to the total weight of the composition.

Oil or organic solvent

The compositions according to the present patent application can also comprise one or more oil(s) or organic solvent(s). The term "oil" is understood to mean a fatty substance which is liquid at ambient temperature and atmospheric pressure. The composition according to the invention can comprise at least one volatile oil and/or at least one non-volatile oil, and their mixtures.

Volatile oil

The composition according to the invention can comprise at least one volatile oil.

The term "volatile oil" is understood to mean an oil (or non-aqueous medium) capable of evaporating on contact with the skin in less than one hour, at ambient temperature and atmospheric pressure. The volatile oil is a volatile cosmetic oil which is liquid at ambient temperature. More specifically, a volatile oil exhibits an evaporation rate of between 0.01 and 200 mg/cm$^2$/min, limits included.

In order to measure this evaporation rate, 15 g of oil or of oil mixture to be tested are introduced into a crystallizing dish with a diameter of 7 cm placed on a balance in a large chamber of approximately 0.3 m$^3$ which is regulated in temperature, at a temperature of 25°C, and regulated in hygrometry, at a relative humidity of 50%. The liquid is allowed to freely evaporate, without stirring it, while providing ventilation by means of a fan (Papst-Motoren, reference 8550 N, rotating at 2700 revolutions per minute) placed in a vertical position above the crystallizing dish containing the said oil or the said mixture, the blades being directed towards the crystallizing dish and at a distance of 20 cm with respect to the bottom of the crystallizing dish. The weight of oil remaining in the crystallizing dish is measured at regular intervals. The evaporation rates are expressed in mg of oil evaporated per unit of surface area (cm$^2$) and per unit of time (minute).

This volatile oil can be a hydrocarbon oil.

The volatile hydrocarbon oil can be chosen from hydrocarbon oils having from 7 to 16 carbon atoms.

The composition according to the invention can comprise one or more volatile branched alkane(s). The term "one or more volatile branched alkane(s)" is understood to mean, without distinction, "one or more volatile branched alkane oil(s)".

Mention may in particular be made, as volatile hydrocarbon oil having from 7 to 16 carbon atoms, of branched $C_8$-$C_{16}$ alkanes, such as $C_8$-$C_{16}$ isoalkanes (also known as isoparaffins), isododecane, isodecane, isohexadecane, for example the oils sold under the Isopar or Permethyl trade names, branched $C_8$-$C_{16}$ esters, such as isohexyl neopentanoate, and their mixtures. Preferably, the volatile hydrocarbon oil having from 8 to 16 carbon atoms is chosen from isododecane, isodecane, isohexadecane and their mixtures, and is in particular isododecane.

The composition according to the invention can comprise one or more volatile linear alkane(s). The term "one or more volatile linear alkane(s)" is understood to mean, without distinction, "one or more volatile linear alkane oil(s)".

A volatile linear alkane which is suitable for the invention is liquid at ambient temperature (approximately 25°C) and at atmospheric pressure (760 mmHg).

A "volatile linear alkane" which is suitable for the invention is understood to mean a cosmetic linear alkane which is capable of evaporating on contact with the skin in less than one hour at ambient temperature (25°C) and atmospheric pressure (760 mmHg, that is to say 101 325 Pa) and which is liquid at ambient temperature, having in particular an evaporation rate ranging from 0.01 to 15 mg/cm$^2$/min at ambient temperature (25°C) and atmospheric pressure (760 mmHg).

The linear alkanes, preferably of vegetable origin, comprise from 7 to 15 carbon atoms, in particular from 9 to 14 carbon atoms and more particularly from 11 to 13 carbon atoms.

Mention may be made, as example of linear alkane suitable for the invention, of the alkanes described in Patent Applications WO 2007/068371 or WO 2008/155059 from Cognis (mixtures of distinct alkanes which differ by at least one carbon). These alkanes are obtained from fatty alcohols, themselves obtained from coconut oil or palm oil.

Mention may be made, as example of linear alkane suitable for the invention, of n-heptane ($C_7$), n-octane ($C_8$), n-nonane ($C_9$), n-decane ($C_{10}$), n-undecane ($C_{11}$), n-dodecane ($C_{12}$), n-tridecane ($C_{13}$), n-tetradecane ($C_{14}$), n-pentadecane ($C_{15}$) and their mixtures, in particular the mixture of n-undecane ($C_{11}$) and n-tridecane ($C_{13}$) described in Example 1 of Patent Application WO 2008/155059 of Cognis. Mention may also be made of n-dodecane ($C_{12}$) and n-tetradecane ($C_{14}$), sold by Sasol respectively under the references Parafol 12-97 and Parafol 14-97, and also their mixtures.

Use may be made of the linear alkane alone or as a mixture of at least two distinct alkanes which differ from one another by a carbon number of at least 1, in particular a mixture of at least two distinct linear alkanes comprising from 10 to 14 carbon atoms which differ from one another by a carbon number of at least 2, especially a mixture of volatile linear $C_{11}$/$C_{13}$ alkanes or a mixture of linear $C_{12}$/$C_{14}$ alkanes, especially an n-undecane/n-tridecane mixture (such a mixture can be obtained according to Example 1 or Example 2 of WO 2008/155059).

In an alternative form or additionally, the composition prepared can comprise at least one volatile silicone oil or solvent which is compatible with a cosmetic use.

The term "silicone oil" is understood to mean an oil comprising at least one silicon atom and in particular comprising Si-O groups. According to one embodiment, the said composition comprises less than 10% by weight of volatile silicone oil(s), with respect to the total weight of the composition, better still less than 5% by weight, indeed is even devoid of silicone oil.

Mention may be made, as volatile silicone oil, of cyclic polysiloxanes, linear polysiloxanes and their mixtures. Mention may be made, as volatile linear polysiloxanes, of hexamethyldisiloxane, octamethyltrisiloxane, decamethyltetrasiloxane, tetradecamethylhexasiloxane and hexadecamethylheptasiloxane. Mention may be made, as volatile cyclic

polysiloxanes, of hexamethylcyclotrisiloxane, octamethylcyclotetrasiloxane, decamethylcyclopentasiloxane and dodecamethylcyclohexasiloxane.

In an alternative form or additionally, the composition prepared can comprise at least one volatile fluoro oil.

The term "fluoro oil" is understood to mean an oil comprising at least one fluorine atom.

Mention may be made, as volatile fluoro oil, of nonafluoromethoxybutane or perfluoromethylcyclopentane, and their mixtures.

A composition according to the invention is preferably devoid of volatile oil. However, at least one volatile oil can be present in a total content ranging from 0.05% to 20 % by weight, with respect to the total weight of the composition, preferably ranging from 0.1% to 15% by weight and preferentially ranging from 0.1% to 10% by weight. In particular, the volatile oil can be present in the composition in a content ranging from 0.5% to 5% by weight, with respect to the total weight of the composition.

According to a preferred embodiment, a composition according to the invention comprises less than 5% by weight of volatile oil(s), with respect to the total weight of the composition.

Non-volatile oil

The composition according to the invention can comprise at least one non-volatile oil.

The term "non-volatile oil" is understood to mean an oil which remains on the skin or the keratinous fibre at ambient temperature and pressure. More specifically, a non-volatile oil exhibits an evaporation rate of strictly less than 0.01 $mg/cm^2/min$.

The said at least one non-volatile oil which is suitable for the present invention can be chosen from hydrocarbon oils and silicone oils.

The non-volatile hydrocarbon oils suitable for the present invention can be chosen in particular from:

- hydrocarbon oils of vegetable origin, such as triglycerides composed of fatty acid esters of glycerol, the fatty acids of which can have varied chain lengths from $C_4$ to $C_{28}$, it being possible for these chains to be saturated or unsaturated and linear or branched; these oils are in particular wheat germ oil, sunflower oil, grape seed oil, sesame oil, maize oil, apricot oil, castor oil, shea oil, avocado oil, olive oil, soybean oil, sweet almond oil, palm oil, rapeseed oil, cottonseed oil, hazelnut oil, macadamia oil, jojoba oil, alfalfa oil, poppy oil, pumpkinseed oil, gourd oil, blackcurrant oil, evening primrose oil, millet oil, barley oil, quinoa oil, rye oil, safflower oil, candlenut oil, passionflower oil or musk rose oil; or alternatively caprylic/capric acid triglycerides, such as those sold by Stéarineries Dubois or those sold under the names Miglyol 810®, 812® and 818® by Sasol;
- synthetic ethers having from 10 to 40 carbon atoms;
- linear or branched hydrocarbons of mineral or synthetic origin, such as petrolatum, polydecenes, hydrogenated poly(iso)butene, such as Parleam, squalane, and their mixtures;
- synthetic esters, such as oils of formula $R_1COOR_2$ in which $R_1$ represents the residue of a linear or branched fatty acid comprising from 1 to 40 carbon atoms and $R_2$ represents a hydrocarbon chain, in particular a branched hydrocarbon chain, comprising from 1 to 40 carbon atoms, provided that $R_1 + R_2 \geq 10$, such as, for example, purcellin oil (cetearyl octanoate), isopropyl myristate, isopropyl palmitate, $C_{12}$ to $C_{15}$ alkyl benzoate, hexyl laurate, diisopropyl adipate, isononyl isononanoate, 2-ethylhexyl palmitate, isostearyl isostearate, or octanoates, decanoates or ricinoleates of alcohols or polyalcohols, such as propylene glycol dioctanoate; hydroxylated esters, such as isostearyl lactate or diisostearyl malate; and pentaerythritol esters;
- fatty alcohols which are liquid at ambient temperature and which comprise a branched and/or unsaturated carbon-based chain having from 12 to 26 carbon atoms, such as octyldodecanol, isostearyl alcohol, oleyl alcohol, 2-hexyldecanol, 2-butyloctanol or 2-undecylpentadecanol; - higher fatty acids, such as oleic acid, linoleic acid, linolenic acid and their mixtures.

The non-volatile silicone oils suitable for the present invention can be chosen in particular from:

- non-volatile polydimethylsiloxanes (PDMSs), polydimethylsiloxanes comprising alkyl or alkoxy groups which are pendent and/or at the end of the silicone chain, which groups each have from 2 to 24 carbon atoms, or phenylated silicones, such as phenyl trimethicones, phenyl dimethicones, phenyl(trimethylsiloxy)diphenylsiloxanes, diphenyl dimethicones, diphenyl(methyldiphenyl)trisiloxanes or (2-phenylethyl)trimethylsiloxysilicates.

A composition according to the invention is preferably devoid of non-volatile oil. However, the total content of non-volatile oil(s) in a composition in accordance with the invention can range from 0.01% to 10% by weight, in particular from 0.1% to 8% by weight and preferably from 0.25% to 5% by weight, with respect to the total weight of the composition.

According to a preferred embodiment, a composition according to the invention comprises less than 5% by

weight of non-volatile oil(s), with respect to the total weight of the composition.

**Emulsifying system**

The compositions according to the invention comprise an emulsifying system.

This emulsifying system comprises a mixture of these three types of surfactants :

- at least one anionic surfactant endowed with a cationic counterion,
- at least one non-ionic surfactant having, at 25°C, an HLB (hydrophilic-lipophilic balance) balance within the Griffin meaning of greater than or equal to 8,
- at least one non-ionic surfactant having, at 25°C, an HLB (hydrophilic-lipophilic balance) balance within the Griffin meaning of less than 8.

This emulsifying system additionally comprises at least one co-surfactant.

Advantageously, the surfactant(s) is (are) distinct from oxyalkylenated wax(es), in particular distinct from PEG-Wax.

Advantageously, the surfactant(s) is (are) coated to particles when introduced in the composition.

*Anionic surfactant(s)*

The anionic surfactant(s) which can be used in the context of the present invention is (are) in particular (non-limiting list) one (of the) salt(s) (especially alkaline salts, in particular sodium salts, ammonium salts, amine salts, aminoalcohol salts or magnesium salts) of the following compounds:

- fatty acids, in particular salts of fatty acids and especially amine salts or alkali metal salts, such as salts of oleic acid, ricinoleic acid, palmitic acid, stearic acid;
- and their mixtures.

Each of these said anionic surfactants can be used alone or as a mixture with one or more other said anionic surfactants.

Use is preferably made, among anionic surfactants, of fatty acids, especially $C_{12}$-$C_{22}$, preferably $C_{14}$-$C_{18}$, fatty acids, and in particular of stearic acid.

In particular, this (these) anionic surfactant(s) in accordance with the invention can be present at a content of greater than or equal to 1% by weight, with respect to the total weight of the composition, in particular ranging from 1.5% to 8% by weight and better still from 2% to 6% by weight, with respect to the total weight of the composition.

*Cationic counterion*

The anionic surfactant comprises at least one cationic counterion.

This cationic counterion can be of inorganic origin, in particular chosen from alkali metal and alkaline earth metal cations, or of organic origin.

The alkali metal cation(s) can be chosen from sodium and potassium.

The alkaline earth metal cation(s) can be chosen from calcium and magnesium.

The cation(s) of organic origin can be chosen from ammonium, and its amine and aminoalcohol derivatives, or magnesium. Preferably, the cation(s) of organic origin is (are) chosen from ammonium, and its amine and aminoalcohol derivatives.

According to a preferred embodiment, the cation is a primary (poly)hydroxyalkylamine.

The term "primary (poly)hydroxyalkylamine" is understood in particular to mean a primary dihydroxyalkylamine, it being understood that the term "primary" is understood to mean a primary amine functional group, i.e. $-NH_2$, and the alkyl group is a linear or branched $C_1$-$C_8$ hydrocarbon chain, preferably a branched $C_4$ hydrocarbon chain, such as 1,3-dihydroxy-2-methylpropyl.

The primary (poly)hydroxyalkylamine is preferentially 1,3-dihydroxy-2-methyl-2-propylamine (also known as aminomethyl propanediol or AMPD).

The total content of cationic counterion(s), in particular of primary (poly)hydroxyalkylamine, especially of AMPD, is preferably greater than or equal to 0.01%, especially inclusively between 0.1% and 4% by weight and better still between 0.5% and 2% by weight, with respect to the total weight of the composition.

An emulsifying system in accordance with the invention comprises at least one non-ionic surfactant.

*Non-ionic surfactant(s) with an HLB of greater than or equal to 8 at 25°C*

A composition in accordance with the invention employs at least one non-ionic surfactant having, at 25°C, an HLB (hydrophilic-lipophilic balance) balance within the Griffin meaning of greater than or equal to 8.
The HLB value according to Griffin is defined in J. Soc. Cosm. Chem., 1954 (volume 5), pages 249-256.
Reference may be made to the Kirk-Othmer Encyclopedia of Chemical Technology, volume 22, pp. 333-432, 3rd edition, 1979, Wiley, for the definition of the emulsifying properties and functions of surface-active agents, in particular pp. 347-377 of this reference.
The non-ionic surfactant(s) with an HLB balance of greater than or equal to 8 are advantageously chosen from:

- oxyethylenated and/or oxypropylenated glycerol ethers which can comprise from 1 to 150 oxyethylene and/or oxypropylene units;
- oxyalkylenated alcohols, in particular oxyethylenated and/or oxypropylenated alcohols, which can comprise, for example, from 1 to 150 oxyethylene and/or oxypropylene units, preferably from 20 to 100 oxyethylene units, in particular ethoxylated fatty alcohols, especially $C_8$-$C_{24}$, preferably $C_{12}$-$C_{18}$, fatty alcohols, such as ethoxylated stearyl alcohol comprising 20 oxyethylene units (CTFA name: steareth-20), such as Brij 78, sold by Uniqema, ethoxylated cetearyl alcohol comprising 30 oxyethylene units (CTFA name: ceteareth-30) and the mixture of $C_{12}$-$C_{15}$ fatty alcohols comprising 7 oxyethylene units (CTFA name: $C_{12-15}$ pareth-7), such as that sold under the name Neodol 25-7® by Shell Chemicals;

- esters of fatty acid, in particular $C_8$-$C_{24}$, preferably $C_{16}$-$C_{22}$, fatty acid, and of polyethylene glycol (or PEG) (which can comprise, for example, from 1 to 150 oxyethylene units), such as PEG-50 stearate and PEG-40 monostearate, sold under the name Myrj 52P® by Uniqema;
- esters of fatty acid, in particular $C_8$-$C_{24}$, preferably $C_{16}$-$C_{22}$, fatty acid, and of oxyethylenated and/or oxypropylenated glycerol ethers (which can comprise, for example, from 1 to 150 oxyethylene and/or oxypropylene units), preferably oxyethylenated glycerol ethers, such as polyoxyethylenated glyceryl monostearate comprising 200 oxyethylene units, sold under the name Simulsol 220 TM® by SEPPIC; polyoxyethylenated glyceryl stearate comprising 30 oxyethylene units, such as the product Tagat S® sold by Goldschmidt, polyoxyethylenated glyceryl oleate comprising 30 oxyethylene units, such as the product Tagat O® sold by Goldschmidt, polyoxyethylenated glyceryl cocoate comprising 30 oxyethylene units, such as the product Varionic LI 13® sold by Sherex, polyoxyethylenated glyceryl isostearate comprising 30 oxyethylene units, such as the product Tagat L® sold by Goldschmidt, and polyoxyethylenated glyceryl laurate comprising 30 oxyethylene units, such as the product Tagat I® from Goldschmidt;
- esters of fatty acid, in particular $C_8$-$C_{24}$, preferably $C_{16}$-$C_{22}$, fatty acid, and of oxyethylenated and/or oxypropylenated sorbitol ethers (which can comprise, for example, from 1 to 150 oxyethylene and/or oxypropylene units), preferably oxyethylenated sorbitol ethers, such as polysorbate 60, sold under the name Tween 60® by Uniqema;
- dimethicone copolyol, such as that sold under the name Q2-5220® by Dow Corning;
- dimethicone copolyol benzoate, such as that sold under the names Finsolv SLB 101® and 201® by Fintex;
- copolymers of propylene oxide and of ethylene oxide, also known as EO/PO polycondensates;
- and their mixtures.

The EO/PO polycondensates are more particularly copolymers composed of polyethylene glycol and polypropylene glycol blocks, such as, for example, polyethylene glycol/polypropylene glycol/polyethylene glycol triblock polycondensates. These triblock polycondensates have, for example, the following chemical structure:

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

in which formula a ranges from 2 to 120 and b ranges from 1 to 100.
The EO/PO polycondensates preferably have a weight-average molecular weight ranging from 1000 to 15 000 and better still ranging from 2000 to 13 000. Advantageously, the said EO/PO polycondensates have a cloud point, at 10 g/l in distilled water, of greater than or equal to 20°C, preferably of greater than or equal to 60°C. The cloud point is measured according to Standard ISO 1065. Mention may be made, as EO/PO polycondensate which can be used according to the invention, of the polyethylene glycol/polypropylene glycol/polyethylene glycol triblock polycondensates sold under the Synperonic® names, such as Synperonic PE/L44® and Synperonic PE/F127®, by ICI.
According to a specific embodiment, a composition in accordance with the invention comprises at least one non-ionic surfactant exhibiting, at 25°C, an HLB balance within the Griffin meaning of greater than or equal to 8 chosen from esters of fatty acid, in particular $C_8$-$C_{24}$, preferably $C_{16}$-$C_{22}$, fatty acid, and of oxyethylenated and/or oxypropylenated glycerol ethers (which can comprise from 1 to 150 oxyethylene and/or oxypropylene units), preferably oxyethylenated glycerol ethers.

The non-ionic surfactant(s) exhibiting, at 25°C, an HLB balance within the Griffin meaning of greater than or equal to 8 may be present at a total content of greater than or equal to 1% by weight, with respect to the total weight of the composition, preferably ranging from 1.5% to 8% by weight and better still from 2% to 5% by weight, with respect to the total weight of the composition;

*Non-ionic surfactant(s) with an HLB of less than 8 at 25°C*

A composition according to the invention employs at least one non-ionic surfactant having, at 25°C, an HLB (hydrophilic-lipophilic balance) balance within the Griffin meaning of less than 8.
The HLB value according to Griffin is defined in J. Soc. Cosm. Chem., 1954 (volume 5), pages 249-256.
Reference may be made to the Kirk-Othmer Encyclopedia of Chemical Technology, volume 22, pp. 333-432, 3rd edition, 1979, Wiley, for the definition of the emulsifying properties and functions of surface-active agents, in particular pp. 347-377 of this reference.
The non-ionic surfactant(s) with an HLB of less than 8 at 25°C are advantageously chosen from:

- saccharide esters and ethers, such as sucrose stearate, sucrose cocoate or sorbitan stearate, and their mixtures, for example Arlatone 2121®, sold by ICI, or Span 65V from Uniqema;
- esters of fatty acids, in particular $C_8$-$C_{24}$, preferably $C_{16}$-$C_{22}$, fatty acids, and of polyol, in particular of glycerol or sorbitol, preferably of glycerol, such as glyceryl stearate, for example sold under the name Tegin M® by Goldschmidt, glyceryl laurate such as the product sold under the name Imwitor 312® by Huls, polyglyceryl-2 stearate, sorbitan tristearate and glyceryl ricinoleate;
- oxyalkylenated alcohols, in particular oxyethylenated and/or oxypropylenated alcohols, which can comprise from 1 to 15 oxyethylene and/or oxypropylene units, in particular ethoxylated $C_8$-$C_{24}$, preferably $C_{12}$-$C_{18}$, fatty alcohols, such as ethoxylated stearyl alcohol comprising 2 oxyethylene units (CTFA name: steareth-2), such as Brij 72, sold by Uniqema;
- a cyclomethicone/dimethicone copolyol mixture, such as sold under the name Q2-3225C® by Dow Corning;
- and their mixture.

According to a specific embodiment, a composition in accordance with the invention comprises at least one non-ionic surfactant exhibiting, at 25°C, an HLB balance within the Griffin meaning of less than 8 chosen from esters of fatty acids, in particular $C_8$-$C_{24}$, preferably $C_{16}$-$C_{22}$, fatty acids, and of polyol, in particular of glycerol or sorbitol, preferably of glycerol.
The non-ionic surfactant(s) exhibiting, at 25°C, an HLB balance within the Griffin meaning of less than 8 may be present at a total content of greater than or equal to 1% by weight, with respect to the total weight of the composition, preferably ranging from 1.5% to 8% by weight and better still from 2% to 5% by weight, with respect to the total weight of the composition.
According to a particularly preferred embodiment, an emulsifying system in accordance with the invention comprises:

- at least one anionic surfactant and one cationic counterion, advantageously at a total content of between 2% and 5% by weight, with respect to the total weight of the composition,
- at least one non-ionic surface-active agent with an HLB balance of greater than or equal to 8 at 25°C, advantageously at least one ester of $C_{16}$-$C_{22}$ fatty acid and of oxyethylenated glycerol ethers, advantageously at a content of between 2% and 5% by weight, with respect to the total weight of the composition, and
- at least one non-ionic surface-active agent with an HLB balance of less than 8 at 25°C, advantageously at least esters of $C_{16}$-$C_{22}$ fatty acids and of polyol, preferably of glycerol, advantageously at a total content of between 2% and 5% by weight, with respect to the total weight of the composition.

Preferably, the total content of surfactant(s) is greater than or equal to 8% by weight, with respect to the total weight of the composition. In particular, the total content of surface-active agent(s) does not exceed 12% by weight, with respect to the total weight of the composition.
According to a preferred embodiment, the cosmetic composition according to the present patent application comprises less than 1% by weight and preferably less than 0.5% by weight of triethanolamine and better still is devoid of triethanolamine.
According to a preferred embodiment, the cosmetic composition according to the present patent application comprises less than 1% by weight and preferably less than 0.5% by weight of triethanolamine stearate and better still is devoid of triethanolamine stearate.
The cream emulsions according to the invention advantageously exhibit a pH preferably varying from 5 to 9

and more preferentially from 7 to 9. This pH can be obtained and/or adjusted in a conventional manner.

*Co-surfactant(s)*

A composition according to the invention comprises one or more co-surfactants.
A composition according to the invention comprises at least one co-surfactant chosen from fatty alcohols comprising from 10 to 26 carbon atoms, better still from 12 to 24 carbon atoms and even better still from 14 to 22 carbon atoms.
The total content of co-surfactant(s) is preferably inclusively between 0.01% and 5% by weight, with respect to the total weight of the composition.

**Film-forming polymers**

The compositions according to the present patent application also comprise at least one hydrophilic or lipophilic film-forming polymer.
In the present patent application, the term "film-forming polymer" is understood to mean a polymer which is capable, by itself alone or in the presence of an additional film-forming agent, of forming a macroscopically continuous deposited layer and preferably a cohesive deposited layer, better still a deposited layer having cohesive and mechanical properties such that the said deposited layer can be isolated and handled in isolation, for example when the said deposited layer is prepared by pouring onto a non-stick surface, such as a Teflon-coated or silicone-coated surface.
The film-forming polymer(s) can be provided as a dispersion in an aqueous medium.
Mention may be made, among the film-forming polymers which can be used in the composition of the present invention, of synthetic polymers, of radical type or of polycondensate type, polymers of natural origin and their mixtures.
Mention may be made, as examples of film-forming polymers, of:

- proteins, such as proteins of vegetable origin, for example wheat or soybean proteins, or proteins of animal origin, such as keratins, for example keratin hydrolysates and sulfonic keratins;
- cellulose polymers, such as hydroxyethylcellulose, hydroxypropylcellulose, methylcellulose, ethylhydroxyethylcellulose or carboxymethylcellulose, and also quaternized cellulose derivatives;
- acrylic polymers or copolymers, such as polyacrylates or polymethacrylates;
- vinyl polymers, such as polyvinylpyrrolidones, copolymers of methyl vinyl ether and of malic anhydride, the copolymer of vinyl acetate and of crotonic acid, copolymers of vinylpyrrolidone and of vinyl acetate, copolymers of vinylpyrrolidone and of caprolactam, or polyvinyl alcohol;
- anionic, cationic, amphoteric or non-ionic chitin or chitosan polymers;
- gum arabic, guar gum, xanthan derivatives or karaya gum;
- alginates and carrageenans;
- glycoaminoglycans, hyaluronic acid and its derivatives;
- shellac resin, sandarac gum, dammars, elemis or copals;
- deoxyribonucleic acid;
- mucopolysaccharides, such as chondroitin sulfates;

and their mixtures.
The film-forming polymer can also be present in the composition in the form of particles dispersed in an aqueous phase, generally known under the name of latex or pseudolatex. The techniques for preparing these dispersions are well known to a person skilled in the art.
Use may be made, as aqueous dispersion of film-forming polymer, of the acrylic dispersions sold under the names Neocryl XK-90®, Neocryl A-1070®, Neocryl A-1090®, Neocryl BT-62®, Neocryl A-1079® and Neocryl A-523® by Avecia-Neoresins, Dow Latex 432® by Dow Chemical, Daitosol 5000 AD® or Daitosol 5000 SJ® by Daito Kasey Kogyo; Syntran 5760® by Interpolymer, Allianz Opt® by Rohm & Haas or the aqueous polyurethane dispersions sold under the names Neorez R-981® and Neorez R-974® by Avecia-Neoresins, Avalure UR-405®, Avalure UR-410®, Avalure UR-425®, Avalure UR-450®, Sancure 875®, Avalure UR-445® and Sancure 2060® by Noveon, Impranil 85® by Bayer, Aquamere H-1511® by Hydromer; the sulfopolyesters sold under the brand name Eastman AQ® by Eastman Chemical Products, vinyl dispersions, such as Mexomer PAM®, aqueous polyvinyl acetate dispersions, such as Vinybran® from Nisshin Chemical or those sold by Union Carbide, aqueous dispersions of vinylpyrrolidone, dimethylaminopropylmethacrylamide and lauryldimethylpropylmethacrylamidoammonium chloride terpolymer, such as Styleze W from ISP, aqueous dispersions of polyurethane/polyacrylic hybrid polymers, such as those sold under the references Hybridur® by Air Products or Duromer® from National Starch, core/shell type dispersions: for example those sold by Atofina under the reference Kynar (core: fluoro, shell: acrylic) or those described in the document US 5 188 899 (core: silica, shell:

silicone), and their mixtures.

The total solids content of "film-forming polymer(s)" of the compositions in accordance with the invention is advantageously greater than or equal to 5% by weight, with respect to the total weight of the composition, better still greater than or equal to 8% by weight, in particular preferentially ranges from 5% to 20% by weight, preferably from 6% to 15% by weight and better still from 8% to 12% by weight, with respect to the total weight of the composition.

A composition in accordance with the invention preferably comprises at least one film-forming polymer chosen from an aqueous dispersion of acrylic polymer, an aqueous dispersion of polyurethane, their derivatives (such as salts and esters) and their mixtures.

Preferably, a composition in accordance with the invention comprises:

- an aqueous dispersion of acrylic polymer,
- an aqueous dispersion of polyurethane,

and their derivatives, in particular their salts and their esters.

*Aqueous dispersion of acrylic polymer*

According to a preferred embodiment of the invention, the aqueous dispersion of particles of acrylic polymer is chosen from:

- (meth)acrylate copolymers, advantageously copolymers resulting from the polymerization of at least one $C_1$-$C_8$, preferably $C_2$, alkyl acrylate monomer and of at least one $C_1$-$C_8$, preferably $C_1$, alkyl (meth)acrylate monomer,
- styrene/(meth)acrylate copolymers, in particular a polymer chosen from the copolymers resulting from the polymerization of at least one styrene monomer and at least one (meth)acrylate monomer.

Use may be made according to the invention, as acrylic polymer in aqueous dispersion, of the acrylate copolymer sold under the name Daitosol 5000 AD by Daito Kasei Kogyo, the styrene/acrylate copolymer sold under the name Joncryl SCX-8211® by BASF or Syntran 5760® by Interpolymer, Allianz Opt® sold by Rohm and Haas, the acrylic polymer sold under the reference Acronal® DS - 6250 by BASF or the acrylic copolymer Joncryl® 95 sold by BASF.

The acrylic polymer(s) can be present in a total amount of solids of greater than or equal to 2% by weight, with respect to the total weight of the composition, better still 3% by weight, indeed even 4% by weight or more. In particular, the acrylic polymer(s) can be present at an amount of solids ranging from 3% to 10% by weight, with respect to the total weight of the said composition, better still ranging from 4% to 8% by weight.

*Aqueous dispersion of polyurethane*

The compositions in accordance with the invention advantageously comprise aqueous dispersions of particles of specific polyurethanes.

The polyurethane is preferably a product of the reaction of:

A) a prepolymer according to the formula:

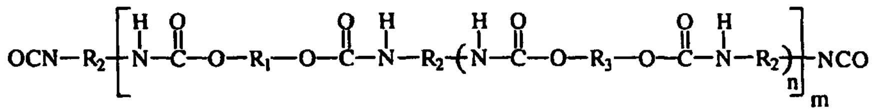

where:

$R_1$ represents a hydrocarbon radical resulting from a polyester polyol and in particular from a polyester diol,
$R_2$ represents a hydrocarbon radical resulting from an aliphatic or cycloaliphatic polyisocyanate,
$R_3$ represents a hydrocarbon radical resulting from a diol, optionally of low molecular weight, optionally substituted by ionic groups,
n has a value from 0 to 5, and
m is > 1;

B) at least one chain extender according to the formula:

$H_2N-R_4-NH_2$

where:

$R_4$ represents an alkylene or alkylene oxide radical unsubstituted by ionic or potentially ionic groups; and

C) at least one chain extender according to the formula:

$H_2N-R_5-NH_2$

where:

$R_5$ represents an alkylene radical substituted by ionic or potentially ionic groups.

Prepolymer A)

The compositions according to the invention comprise aqueous dispersions of specific polyurethanes. Such compositions can be used in cosmetic applications, for making up or caring for the eyelashes or eyebrows.

*Radical R1*

Compounds appropriate for providing the polyhydroxyl, preferably dihydroxyl, radical $R_1$ are polyester polyols, preferably polyester diols, and their mixtures. These compounds are thus advantageously divalent, preferably two hydroxyl groups.

Such compounds can exhibit number-average molecular weights from approximately 700 to approximately 16 000 and preferably from approximately 750 to approximately 5000.

The polyester diol(s) can generally be prepared from:

- aliphatic, cycloaliphatic or aromatic dicarboxylic or polycarboxylic acids or their anhydrides, and

- dihydric alcohols, such as diols chosen from aliphatic, alicyclic or aromatic diols.

The aliphatic dicarboxylic or polycarboxylic acids can be chosen from: succinic acid, fumaric acid, glutaric acid, 2,2-dimethylglutaric acid, adipic acid, itaconic acid, pimelic acid, suberic acid, azelaic acid, sebacic acid, maleic acid, malonic acid, 2,2-dimethylmalonic acid, nonanedicarboxylic acid, decanedicarboxylic acid, dodecanedioic acid, 1,3-cyclohexanedicarboxylic acid, 1,4-cyclohexanedicarboxylic acid, 2,5-norbornanedicarboxylic acid, diglycolic acid, thiodipropionic acid, 2,5-naphthalenedicarboxylic acid, 2,6-naphthalenedicarboxylic acid, phthalic acid, terephthalic acid, isophthalic acid, oxanic acid, o-phthalic acid, tetrahydrophthalic acid, hexahydrophthalic acid or trimellitic acid.

The acid anhydrides can in particular be chosen from o-phthalic, trimellitic or succinic anhydride or a mixture of these.

Preferably, the preferred dicarboxylic acid is adipic acid.

The dihydric alcohols can be chosen from ethanediol, ethylene glycol, diethylene glycol, triethylene glycol, trimethylene glycol, tetraethylene glycol, 1,2-propanediol, dipropylene glycol, tripropylene glycol, tetrapropylene glycol, 1,3-propanediol, 1,4-butanediol, 1,3-butanediol, 2,3-butanediol, 1,5-pentanediol, 1,6-hexanediol, 2,2-dimethyl-1,3-propanediol, 1,4-dihydroxycyclohexane, 1,4-dimethylolcyclohexane, cyclohexanedimethanol, 1,8-octanediol, 1,10-decanediol, 1,12-dodecanediol, neopentyl glycol or their mixtures. Cycloaliphatic and/or aromatic dihydroxyl compounds are also, of course, suitable as dihydric alcohol(s) for the preparation of polyester polyol(s).

The polyester diols can also be chosen from lactone homopolymers or copolymers, which are preferably obtained via addition reactions of lactones or mixtures of lactones, such as butyrolactone, ε-caprolactone and/or methyl-ε-caprolactone, with suitable polyfunctional, preferably difunctional, initiator molecules, such as, for example, the dihydric alcohols mentioned above. The corresponding ε-caprolactone polymers are preferred.

The polyester polyol, preferably polyester diol, radical $R_1$ can advantageously be obtained by polycondensation of dicarboxylic acids, such as adipic acid, with polyols, in particular diols, such as hexanediol or neopentyl glycol, and their mixtures.

*Radical R2*

Polyisocyanates appropriate for providing the hydrocarbon radical $R_2$ comprise organic diisocyanates exhibiting a molecular weight from approximately 112 to 1000 and preferably from approximately 140 to 400.

Preferred diisocyanates are those represented by the general formula $R_2(NCO)_2$ indicated above, in which $R_2$ represents a divalent aliphatic hydrocarbon group comprising from 4 to 18 carbon atoms, a divalent cycloaliphatic

hydrocarbon group comprising from 5 to 15 carbon atoms, a divalent arylaliphatic hydrocarbon group comprising from 7 to 15 carbon atoms or a divalent aromatic hydrocarbon group comprising from 6 to 15 carbon atoms. Examples of organic diisocyanates which are suitable comprise tetramethylene diisocyanate, 1,6-hexamethylene diisocyanate, dodecamethylene diisocyanate, 1,3-cyclohexane diisocyanate and 1,4-cyclohexane diisocyanate, 1-isocyanato-3-isocyanatomethyl-3,5,5-trimethylcyclohexane (isophorone diisocyanate or IPDI), bis(4-isocyanatocyclohexyl)methane, 1,3-bis(isocyanatomethyl)cyclohexane and 1,4-bis(isocyanatomethyl)cyclohexane, and bis(4-isocyanato-3-methylcyclohexyl)methane. Mixtures of diisocyanates can, of course, be used. Preferred diisocyanates are aliphatic and cycloaliphatic diisocyanates. 1,6-Hexamethylene diisocyanate, isophorone diisocyanate and dicyclohexylmethane diisocyanate, and their mixtures, are particularly preferred.

*Radical R3*

The use of diols, in particular low molecular weight diols, $R_3$ can make possible stiffening of the polymer chain and is optional. The expression "low molecular weight diols" means diols exhibiting a molecular weight from approximately 62 to 700, preferably from 62 to 200. They can comprise aliphatic, alicyclic or aromatic groups. The preferred compounds comprise only aliphatic groups. The diols used preferably exhibit up to 20 carbon atoms and can be chosen from ethylene glycol, diethylene glycol, 1,2-propanediol, 1,3-propanediol, 1,4-butanediol, 1,3-butylene glycol, neopentyl glycol, butylethylpropanediol, cyclohexanediol, 1,4-cyclohexanedimethanol, 1,6-hexanediol, bisphenol A (2,2-bis(4-hydroxyphenyl)propane), hydrogenated bisphenol A (2,2-bis(4-hydroxycyclohexyl)propane) and their mixtures. Preferably, $R_3$ results from neopentyl glycol.

Optionally, the low molecular weight diols can comprise ionic or potentially ionic groups. Suitable low molecular weight diols comprising ionic or potentially ionic groups are those disclosed in Patent US 3 412 054. Preferred compounds comprise dimethylolbutanoic acid (DMBA), dimethylolpropionic acid (DMPA) and carboxyl-containing caprolactone-polyester diol. If low molecular weight diols comprising ionic or potentially ionic groups are used, they are preferably used in an amount such that < 0.30 meq. of COOH is present per gram of polyurethane in the polyurethane dispersion. Preferably, low molecular weight diols comprising ionic or potentially ionic groups are not used.

The prepolymer chain is extended using two categories of chain extender, B) and C).

*B) Chain extenders*

Compounds B) of the first category of chain extender exhibit the formula:

$$H_2N-R_4-NH_2$$

where $R_4$ represents an alkylene or alkylene oxide radical unsubstituted by ionic or potentially ionic groups.

Thus, the chain extender can be chosen from:

- alkylenediamines, such as hydrazine, ethylenediamine, propylenediamine, 1,4-butylenediamine and piperazine;
- alkylene oxide diamines, such as dipropylamine-diethylene glycol (DPA-DEG available from Tomah Products, Milton, WI), 2-methyl-1,5-pentanediamine (Dytec A from DuPont), hexanediamine, isophoronediamine and 4,4-methylenebis(cyclohexylamine), and the series of DPA-etheramines available from Tomah Products, Milton, WI, comprising dipropylamine-propylene glycol, dipropylamine-dipropylene glycol, dipropylamine-tripropylene glycol, dipropylamine-poly(propylene glycol), dipropylamine-ethylene glycol, dipropylamine-poly(ethylene glycol), dipropylamine-1,3-propanediol, dipropylamine-2-methyl-1,3-propanediol, dipropylamine-1,4-butanediol, dipropylamine-1,3-butanediol, dipropylamine-1,6-hexanediol and dipropylamine-cyclohexane-1,4-dimethanol, and their mixtures.

Preferably, the chain extender B) is chosen from ethylenediamine, diethanolamine and their mixtures.

*C) Chain extenders*

The second category of chain extenders are compounds C) exhibiting the formula:

$$H_2N-R_5-NH_2$$

where $R_5$ represents an alkylene radical substituted by ionic or potentially ionic groups. These compounds exhibit an ionic or potentially ionic group and two groups which are reactive with isocyanate groups. The ionic group or the potentially ionic group can be chosen from the group consisting of tertiary or quaternary ammonium groups, groups which can be converted into such a group, a carboxyl group, a carboxylate group, a sulfonic acid group and a sulfonate group. The

said at least partial conversion of the groups which can be converted into salt groups of the type mentioned can take place before or during the mixing with water. Specific compounds comprise diaminosulfonates, such as, for example, the sodium salt of N-(2-aminoethyl)-2-aminoethanesulfonic acid (AAS) or the sodium salt of N-(2-aminoethyl)-2-aminopropionic acid.

Preferably, $R_5$ represents an alkylene radical substituted by sulfonic acid or sulfonate groups.

Preferably, this compound is the sodium salt of N-(2-aminoethyl)-2-aminoethanesulfonic acid (AAS).

*Chain terminators*

The polyurethane according to the invention can also comprise compounds which are located in each case at the ends of the chains and which terminate the said chains. These chain terminators can derive from compounds exhibiting the formula:

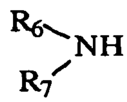

in which $R_6$ represents a hydrogen atom or an alkylene radical optionally exhibiting a hydroxyl end and $R_7$ represents an alkylene radical optionally exhibiting a hydroxyl end. Suitable compounds comprise compounds such as monoamines, in particular secondary monoamines, or monoalcohols. Examples comprise: methylamine, ethylamine, propylamine, butylamine, octylamine, laurylamine, stearylamine, isononyloxypropylamine, dimethylamine, diethylamine, dipropylamine, dibutylamine, N-methylaminopropylamine, diethyl(methyl)aminopropylamine, morpholine, piperidine, diethanolamine and suitable substituted derivatives of the latter, amide-amines of primary diamines and of monocarboxylic acids, monoketimes of primary diamines, primary/tertiary amines, such as N,N-dimethylaminopropylamine, and the like. The chain-terminator alcohols can be chosen from $C_1$-$C_{10}$ alcohols, such as methanol, butanol, hexanol, 2-ethylhexyl alcohol and isodecyl alcohol, and their mixtures. Aminoalcohols, such as aminomethylpropanol (AMP), are also suitable.

In one embodiment of the invention, diethylene glycol is used to obtain the polyurethane, either as a low molecular weight diol or as part of the non-ionic chain extender via the use of dipropylamine-diethylene glycol. In the case where diethylene glycol is used as low molecular weight diol, then, preferably, DPA-DEG is not used as non-ionic chain extender. Analogously, if DPA-DEG is used as non-ionic chain extender, then, preferably, diethylene glycol is not used as low molecular weight diol.

*Preparation processes*

The compositions according to the invention comprise an aqueous polyurethane dispersion suitable for use in products for making up or caring for keratinous fibres, such as the eyelashes or eyebrows, and are capable of being obtained by a preparation process comprising the following stages:

A) the preparation of an aqueous polyurethane dispersion by

1) the formation of a prepolymer having isocyanate functionality by reacting:

1a) a polyester polyol and in particular a polyester diol,
1b) an aliphatic or cycloaliphatic polyisocyanate, and
1c) a low molecular weight diol, optionally substituted by ionic groups;

2) an extension of the prepolymer chain by:

2a) at least one chain extender according to the formula:

$$H_2N\text{-}R_4\text{-}NH_2$$

where $R_4$ represents an alkylene or alkylene oxide radical unsubstituted by ionic or potentially ionic groups, and

2b) at least one chain extender according to the formula:

$$H_2N\text{-}R_5\text{-}NH_2$$

where $R_5$ represents an alkylene radical substituted by ionic or potentially ionic groups,

in the presence of an organic solvent, to form a polyurethane;
3) the dispersion of the polyurethane in water; and
4) the removal of the organic solvent, which makes it possible to obtain an aqueous polyurethane dispersion; and

the mixing of the polyurethane dispersion with water or ethanol.

More particularly, a process for producing a polyurethane dispersion suitable for use in make-up products can comprise the following stages: a) the reaction in a first stage of at least one polyester polyol compound and of a low molecular weight diol optionally substituted by an ionic group (dihydroxyl compounds) with diisocyanate to form the prepolymer A), then b) the dissolution in a second stage of the prepolymer in an organic solvent, c) the reaction in a third stage of the prepolymer solution comprising the isocyanate with the two categories of chain extender and, optionally, the chain terminator, d) the formation in a fourth stage of the dispersion by addition of water, and e) the removal in a fifth stage of the organic solvent.

The free sulfonic acid groups incorporated are neutralized between the third and fourth stages. Suitable neutralizing agents included are primary, secondary or tertiary amines. Preference is given, among these, to trialkyl-substituted tertiary amines. Examples of these amines are trimethylamine, triethylamine, triisopropylamine, tributylamine, N,N-dimethylcyclohexylamine, N,N-dimethylstearylamine, N,N-dimethylaniline, N-methylmorpholine, N-ethylmorpholine, N-methylpiperazine, N-methylpyrrolidine, N-methylpiperidine, N,N-dimethylethanolamine, N,N-diethylethanolamine, triethanolamine, N-methyldiethanolamine, dimethylaminopropanol, 2-methoxyethyldimethylamine, N-hydroxyethylpiperazine, 2-(2-dimethylaminoethoxy)ethanol and 5-diethylamino-2-pentanone. The preferred tertiary amines are those which do not comprise active hydrogen(s) as determined by the Zerewitinoff test, given that hydrogen can react with the isocyanate groups of the prepolymers, which can bring about gelling, the formation of insoluble particles or chain termination.

The polyurethane dispersions can be produced by what is known as the acetone process. In the acetone process, the synthesis of the aqueous polyurethane preparations on which the dispersions according to the invention are based is carried out in a multi-stage process.

In a first stage, a prepolymer comprising isocyanate groups is synthesized from the polyester polyol compound, the diisocyanate and the low molecular weight diol. The amounts of individual components are calculated so that the isocyanate content of the prepolymers is between 1.4% and 5.0% by weight, preferably between 2.0% and 4.5% by weight and particularly preferably between 2.6% and 4.0% by weight. The low molecular weight diol is present in an amount of 0 eq.% to 80 eq.%, on the basis of the amount of NCO equivalents, and preferably of 0 eq.% to 10 eq.%.

The prepolymer obtained exhibits the structure:

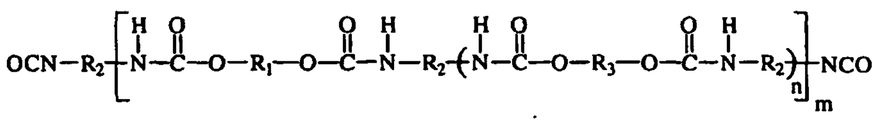

where:

$R_1$ represents a polyester polyol and in particular a polyester diol,
$R_2$ represents a hydrocarbon radical or an aliphatic or cycloaliphatic polyisocyanate,
$R_3$ represents a radical of a low molecular weight diol, optionally substituted by ionic groups,

n has a value <5, and
m has a value >1.

Preferably, n has a value from 1 to 3 and m has a value from 1 to 5.

In a second stage, the prepolymer produced in stage 1 is dissolved in an organic solvent, at least partially miscible with water, not comprising groups which react with isocyanate. The preferred solvent is acetone. Other solvents, such as, for example, 2-butanone, tetrahydrofuran or dioxane, or the mixtures of these solvents, can, however, also be used. The amounts of solvent to be used must be calculated so that a solids content of 25% to 60% by weight, preferably of 30% to 50% by weight and particularly preferably of 35% to 45% by weight is obtained.

In a third stage, the solution of prepolymer comprising isocyanate is reacted with mixtures of chain extenders having amino functionality and, optionally, chain terminators, to form the high molecular weight polyurethane. Sufficient amounts of chain extenders and of chain terminators are used so that the calculated number-average molecular weight (Mn) of the polyurethane obtained is between 10 000 and 100 000 daltons and preferably between 10 000 and 50 000

daltons. The non-ionic chain extender is present in an amount of 15 eq.% to 90 eq.%, preferably of 35.0 eq.% to 55 eq.%, on the basis of the residual amount of NCO equivalents present in the prepolymer. The ionic chain extender is present in an amount of 10 eq.% to 50 eq.%, preferably of 25 eq.% to 35 eq.%, on the basis of the residual amount of NCO equivalents present in the prepolymer. The chain terminator is present in an amount of 0 eq.% to 35 eq.%, preferably of 20 eq.% to 30 eq.%, on the basis of the residual amount of NCO equivalents present in the prepolymer.

In a fourth stage, the high molecular weight polyurethane is dispersed in the form of a dispersion having fine particles by the addition of water to the solution or of the solution to water.

In a fifth stage, the organic solvent is partially or totally removed by distillation, optionally under reduced pressure. The amount of water in the fourth stage is calculated so that the aqueous polyurethane dispersions according to the invention display a solids content of 20% to 60% by weight and preferably of 28% to 42% by weight.

Such polyurethane dispersions are, for example, Polyurethane 34, sold under the trade names Baycusan C1000 and C1001 by Bayer, and Polyurethane 35, sold under the trade name Baycusan C1004 by Bayer.

The polyurethane(s) can be present in a total amount of solids of greater than or equal to 2% by weight, with respect to the total weight of the composition, better still 4 % by weight, indeed even 10 % by weight or more. The polyurethane can, for example, be present in an amount of solids ranging from 3.5% to 15% by weight, with respect to the total weight of the said composition, better still ranging from 4% to 12% by weight.

The film-forming polymer(s) and the wax(es) are advantageously present in the composition at a respective total content of solids such that the ratio by weight of the film-forming polymer(s) to the wax(es) is greater than or equal to 1, in particular greater than or equal to 1.2, preferably inclusively between 1.1 and 4, more preferably still between 1.2 and 3.5.

The film-forming polymer(s) and the surfactant(s) are advantageously present in the composition at a respective total content of solids such that the ratio by weight of the film-forming polymer(s) to the surfactant(s) is less than or equal to 3, in particular less than or equal to 2, more preferably less than or equal to 1, preferably inclusively between 0.5 and 3.5, more preferably still between 0.6 and 0.9.

The film-forming polymer(s) plus the wax(es) and the surfactant(s) are advantageously present in the composition at a respective total content of solids such that the ratio by weight of the film-forming polymer(s) plus the wax(es) to the surfactant(s) is less than or equal to 3, preferably less than or equal to 2, in particular between 1 and 2.75.

## Organic lipophilic gelling agents

A composition according to the invention comprises at least one organic lipophilic gelling agent. Thus, a composition according to the invention advantageously does not comprise inorganic lipophilic gelling agent and in particular does not comprise clay and fumed silica optionally hydrophobically surface-treated, the size of the particles of which is less than 1 $\mu$m, and their derivatives.

The gelling agent(s) which can be used can be polymeric or molecular organic lipophilic gelling agents.

The compositions in accordance with the invention comprise, as organic lipophilic gelling agent, a polycondensate of hydrocarbon polyamide type.

The term "hydrocarbon polyamide" is understood to mean a polyamide formed essentially of, indeed even consisting of, carbon and hydrogen atoms, and optionally of oxygen or nitrogen atoms, and not comprising a silicon or fluorine atom. It can comprise alcohol, ester, ether, carboxylic acid, amine and/or amide groups.

The polymeric organic lipophilic gelling agent(s), are, for example, chosen from polycondensates of polyamide type resulting from the condensation between ($\alpha$) at least one acid chosen from dicarboxylic acids comprising at least 32 carbon atoms, such as fatty acid dimers, and ($\beta$) an alkylenediamine and in particular ethylenediamine, in which the polyamide polymer comprises at least one end carboxylic acid group esterified or amidated with at least one monoalcohol or one monoamine comprising from 12 to 30 carbon atoms, which are linear and saturated, in particular ethylenediamine/stearyl dilinoleate copolymers, such as that sold under the name Uniclear 100 VG® by Arizona Chemical;

Advantageously, this polycondensate exhibits a weight-average molecular weight of less than 100 000 g/mol, in particular ranging from 1000 to 100 000 g/mol, especially of less than 50 000 g/mol, in particular ranging from 1000 to 50 000 g/mol and more particularly ranging from 1000 to 30 000 g/mol, preferably from 2000 to 20 000 g/mol and better still from 2000 to 10 000 g/mol.

This polyamide is insoluble in water, in particular at 25°C.

According to a specific embodiment of the invention, the polyamide is a polyamide comprising an amide ester terminator of formula (I)

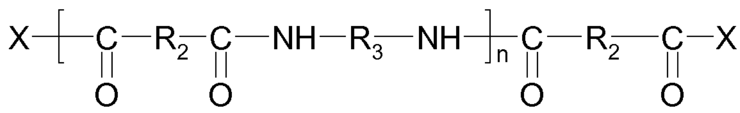
(I)

in which X represents an $-OR_1$ group in which $R_1$ is a linear or branched $C_8$ to $C_{22}$, preferably $C_{16}$ to $C_{22}$, alkyl radical which can be identical or different, $R_2$ is a $C_{28}$-$C_{42}$ dimer diacid residue, $R_3$ is an ethylenediamine radical and n is between 2 and 5.

Mention may be made, as polyamide compounds of formula (I), of the commercial products sold by Arizona Chemical under the names Uniclear 80 and Uniclear 100 or also Uniclear 80 V, Uniclear 100 V and Uniclear 100 VG, the INCI name of which is "ethylenediamine/stearyl dimer dilinoléate copolymer". They are sold, respectively, in the form of a gel comprising 80% of active material in a mineral oil and comprising 100% of active material. They have a softening point of 88 to 94°C. These commercial products are a mixture of copolymers of a $C_{36}$ diacid condensed with ethylenediamine, with a weight-average molecular weight of approximately 6000 g/mol. The terminal ester groups result from the esterification of the remaining acid terminators with cetyl alcohol, stearyl alcohol or their mixtures (also known as cetylstearyl alcohol).

Preferably, the total amount of lipophilic gelling agent(s) and in particular of polycondensate of the hydrocarbon polyamide type in the compositions in accordance with the invention is inclusively between 0.5% and 6% by weight, on a dry basis, preferably between 0.75% and 4% by weight or better still between 1% and 2.5% by weight, with respect to the total weight of the composition.

It should be noted that the organic lipophilic gelling agent(s) make(s) it possible, when a high content of film-forming polymer(s) (in particular of greater than or equal to 5% by weight, with respect to the total weight of the composition) is (are) employed, to relax the cosmetic composition in accordance with the invention and thus prevent(s) this composition from excessively hardening, thus facilitating its removal and its application.

The film-forming polymer(s) and the organic lipophilic gelling agent(s), in particular the polycondensate(s) of hydrocarbon polyamide type, is (are) advantageously present in the composition at a respective total solids content such that the ratio by weight of the film-forming polymer(s) to the organic lipophilic gelling agent(s) is preferably between 3 and 6, more preferably still between 4 and 5.

## Hydrophilic gelling agents

The compositions according to the present patent application can also comprise at least one hydrophilic gelling agent and they can be chosen from:

- homo- or copolymers of acrylic or methacrylic acid or their salts and their esters and in particular the products sold under the names Versicol F® or Versicol K® by Allied Colloid or Ultrahold 8® by Ciba-Geigy or polyacrylic acids of Synthalen K type,
- copolymers of acrylic acid and of acrylamide sold in the form of their sodium salt under the names Reten® by Hercules and the sodium salts of polyhydroxycarboxylic acids sold under the name Hydagen F® by Henkel,
- polyacrylic acid/alkyl acrylate copolymers of Pemulen type,
- AMPS (polyacrylamidomethylpropanesulfonic acid partially neutralized with aqueous ammonia and highly crosslinked), sold by Clariant,
- AMPS/acrylamide copolymers of Sepigel® or Simulgel® type sold by Seppic, and
- AMPS/polyoxyethylenated alkyl methacrylate copolymers (crosslinked or non-crosslinked), and their mixtures,
- associative polymers and in particular associative polyurethanes, such as the $C_{16}$-$OE_{120}$-$C_{16}$ polymer from Servo Delden (sold under the name SER AD FX1100, which molecule has a urethane functional group and a weight-average molecular weight of 1300), OE being an oxyethylene unit, Rheolate 205, having a urea functional group, sold by Rheox, or also Rheolate 208 or 204 (these polymers being sold in pure form) or DW 1206B from Rohm & Haas, having a $C_{20}$ alkyl chain and having a urethane bond, sold at 20% of active material in water. It is also possible to use solutions or dispersions of these associative polyurethanes, in particular in water or in aqueous/alcoholic medium. Mention may be made, as examples of such polymers, of SER AD FX1010, SER AD FX1035 and SER AD 1070 from Servo Delden and Rheolate 255, Rheolate 278 and Rheolate 244, sold by Rheox. It is also possible to use the products DW 1206F and DW 1206J and also Acrysol RM 184 or Acrysol 44 from Rohm & Haas or also Borchigel LW 44 from Borchers,

  - and their mixtures.

Some water-soluble film-forming polymers mentioned above can also act as water-soluble gelling agent.

The hydrophilic gelling agents can be present in the compositions according to the invention in a content ranging from 0.05% to 10% by weight, with respect to the total weight of the composition, preferably from 0.1% to 8% by weight and better still from 0.5% to 5% by weight.

## Colorants

The compositions in accordance with the invention comprise at least one colorant.

This (or these) colorant(s) is (are) preferably chosen from pulverulent colorants, fat-soluble dyes, water-soluble dyes and their mixtures.

Preferably, the compositions according to the invention comprise at least one pulverulent colorant. The pulverulent colorants can be chosen from pigments and pearlescent agents, preferably from pigments.

The pigments can be white or coloured, inorganic and/or organic and coated or uncoated. Mention may be made, among the inorganic pigments, of metal oxides, in particular titanium dioxide, optionally surface-treated, zirconium, zinc or cerium oxide, and also iron, titanium or chromium oxide, manganese violet, ultramarine blue, chromium hydrate and ferric blue. Mention may be made, among the organic pigments, of carbon black, pigments of D & C type and lakes based on cochineal carmine of barium, strontium, calcium or aluminium.

The pearlescent agents can be chosen from white pearlescent pigments, such as mica covered with titanium dioxide or with bismuth oxychloride, coloured pearlescent pigments, such as titanium oxide-coated mica with iron oxides, titanium oxide-coated mica with in particular ferric blue or chromium oxide, or titanium oxide-coated mica with an organic pigment of the abovementioned type, and also pearlescent pigments based on bismuth oxychloride.

The fat-soluble dyes are, for example, Sudan red, D&C Red 17, D&C Green 6, β-carotene, soybean oil, Sudan brown, D&C Yellow 11, D&C Violet 2, D&C Orange 5, quinoline yellow and annatto.

Preferably, the pigments present in the compositions according to the invention are chosen from metal oxides.

These colorants can be present in a content ranging from 0.01% to 30% by weight, with respect to the total weight of the composition, in particular from 3% to 15% by weight, with respect to the total weight of the composition.

Preferably, the colorant(s) is (are) chosen from one or more metal oxides present at a content of greater than or equal to 2% by weight, with respect to the total weight of the composition, advantageously inclusively between 3% and 15% by weight, with respect to the total weight of the composition.

## Fillers

The compositions in accordance with the invention advantageously comprise at least one filler.

The fillers can be chosen from those well known to a person skilled in the art and commonly used in cosmetic compositions.

Preferably, a composition in accordance with the invention comprises at least one inorganic filler. Preferably, this (or these) inorganic filler(s) is (are) chosen, for example, from talc, mica, synthetic fluorphlogopite, a hydrophilic clay, such as smectite, and their mixtures.

The fillers, which are preferably inorganic, can represent from 0.5% to 8% by weight, in particular from 2% to 5% by weight, with respect to the total weight of the composition.

## Fibres

The compositions in accordance with the invention can also comprise at least one fibre which makes it possible to improve the lengthening effect.

The term "fibre" should be understood as meaning an object with a length L and a diameter D such that L is very much greater than D, D being the diameter of the circle in which the cross section of the fibre lies. In particular, the ratio L/D (or aspect ratio) is chosen within the range from 3.5 to 2500, in particular from 5 to 500 and more particularly from 5 to 150.

The fibres which can be used in the composition of the invention can be inorganic or organic fibres of synthetic or natural origin. They can be short or long, individual or organized, for example braided, and hollow or solid. They can have any shape and can in particular have a circular or polygonal (square, hexagonal or octagonal) cross section, depending on the specific application envisaged. In particular, their ends are blunted and/or polished to prevent injury.

In particular, the fibres have a length ranging from 1 μm to 10 mm, in particular from 0.1 mm to 5 mm and more particularly from 0.3 mm to 3.5 mm. Their cross section can be included in a circle with a diameter ranging from 2 nm to 500 μm, in particular ranging from 100 nm to 100 μm and more particularly ranging from 1 μm to 50 μm. The weight or count of fibres is often given in denier or decitex and represents the weight in grams per 9 km of yarn. The fibres according to the invention can in particular have a count chosen within the range from 0.15 to 30 denier and in particular

from 0.18 to 18 denier.

The fibres which can be used in the composition of the invention can be chosen from rigid or non-rigid fibres and they can be inorganic or organic fibres of synthetic or natural origin.

Moreover, the fibres may or may not be surface-treated, may or may not be coated and may or may not be coloured.

Mention may be made, as fibres which can be used in the composition according to the invention, of non-rigid fibres, such as polyamide (Nylon®) fibres, or rigid fibres, such as polyimideamide fibres, for example those sold under the Kermel® and Kermel Tech® names by Rhodia, or poly(p-phenylene terephthalamide) (or aramid) fibres, sold in particular under the Kevlar ® name by DuPont de Nemours.

The fibres can be present in the composition according to the invention in a content ranging from 0.01% to 10% by weight, with respect to the total weight of the composition, in particular from 0.1% to 5% by weight and more particularly from 0.3% to 3% by weight.

## Cosmetic active agents

The compositions in accordance with the invention can also comprise at least one cosmetic active agent.

Mention may in particular be made, as cosmetic active agents which can be used in the compositions in accordance with the invention, of antioxidants, preservatives, fragrances, neutralizing agents, emollients, coalescence agents, plasticizers, moisturizing agents, vitamins and screening agents, in particular sunscreens, and their mixtures.

Of course, a person skilled in the art will take care to choose the optional additional additives and/or their amounts so that the advantageous properties of the composition according to the invention are not, or not substantially, detrimentally affected by the envisaged addition.

Preferably, the composition according to the invention is a leave-in composition. Advantageously, the composition is a make-up composition and in particular a mascara.

The following examples are given by way of illustration of the present invention and cannot limit the scope thereof.

## ASSEMBLY

An assembly for coating keratinous fibres suitable for the invention can comprise an applicator configured in order to apply the said cosmetic composition for coating keratinous fibres and, where appropriate, a packaging device suitable for receiving the said composition.

## Applicator

The applicator comprises means which make it possible to smooth and/or separate keratinous fibres, such as the eyelashes or eyebrows, in particular in the form of teeth, bristles or other protruding elements.

The applicator is designed in order to apply the composition to the eyelashes or eyebrows and can comprise, for example, a brush or a comb.

The applicator can also be used for finishing the make-up, over a region of the eyelashes or eyebrows which is made up or laden with the composition.

The brush can comprise a twisted core and bristles held between the turns of the core or can be made in yet another way.

The comb is, for example, produced from a single part by moulding plastic.

In some implementational examples, the application element is mounted at the end of a stem which can be flexible, which can contribute to improving the comfort during application.

## Packaging device

The packaging device comprises a container intended to house the composition for coating keratinous fibres. This composition can then be withdrawn from the container by immersing the applicator in it.

This applicator can be integral with an element for closing the container. This closing element can form a member for grasping the applicator. This grasping member can form a cap to be removably mounted on the said container by any suitable means, such as screwing, snap-fastening, push-fitting or other. Such a container can thus reversibly house the said applicator.

This container can optionally be equipped with a wiper suitable for removing a surplus of product withdrawn by the applicator.

A method for applying the composition according to the invention to the eyelashes or eyebrows can also comprise the following stages:

- forming a deposited layer of the cosmetic composition on the eyelashes or eyebrows,
- leaving the deposited layer on the eyelashes or eyebrows, it being possible for the deposited layer to dry.

It should be noted that, according to another embodiment, the applicator can form a product container. In such a case, a container can, for example, be provided in the grasping member and an internal channel can internally connect this grasping member to the protruding application elements.
Finally, it should be noted that the packaging and application assembly can be provided in the form of a kit, it being possible for the applicator and the packaging device to be housed separately in one and the same packaging article.

## EXAMPLE

1/ Preparation of a mascara composition according to the invention

| Ingredients | Content as % (by total weight of starting material) |
|---|---|
| Glyceryl PEG-200 monostearate | 2.2 |
| Ethylene/vinyl acetate copolymer[1] | 2 |
| Ethyl alcohol | 1 |
| Water | q.s. for 100 |
| Hydrogenated $C_{36}$ diacid /ethylenediamine condensate, esterified by stearyl alcohol[2] | 2 |
| Propylene glycol | 4.4 |
| Cetyl alcohol | 3 |
| Acrylic and styrene copolymer as a 40% aqueous emulsion in a water/butylene glycol/protected sodium lauryl ether sulfate mixture[3] | 11 |
| Black iron oxide | 6 |
| Beeswax | 4 |
| Polyurethane-35[4] | 10 |
| Synthetic fluorphlogopite | 4 |
| Stearic acid | 2.9 |
| Glycerol | 0.5 |
| Xanthan gum | 0.1 |
| Aminomethyl propanediol | 0.8 |
| Glyceryl stearate | 3.3 |
| Carnauba wax | 1.8 |
| Hydroxyethylcellulose | 0.5 |
| Preserving system | q.s. |
| [1]Coolbind 34-1300® from National Starch<br>[2]Uniclear 100 VG® from Arizona Chemical<br>[3]Syntran 5760 CG® from Interpolymer<br>[4]Baycusan C1004® from Bayer | |

These compositions were prepared as follows:

*i. Preparation of the fatty phase*

All the starting materials used are carefully weighed out using a balance (accuracy 0.01 g).
The various waxes are melted in a 500 ml jacketed heating pan with circulation of hot oil to control the temperature. The mixture is heated to approximately 85-90°C.
Once the waxes have melted, homogenization is carried out by stirring using a Moritz stirrer, which is a stirrer of rotor-stator type. It is composed of a stationary part within which a second mobile part rotates at variable speed; this device is used to prepare emulsions since it makes possible very high shearing.
When the waxes are molten and homogenized, the pigments are added and then homogenization is again carried out under the Moritz stirrer. The temperature is then checked (by means of a probe) and adjusted to 85-90°C, if necessary.

*ii. Preparation of the aqueous phase*

Use is made of a high-sided 600 ml beaker, which is weighed empty beforehand, in order to adjust the weight of water before emulsification. The amount of water necessary, preheated in an electric kettle, is stirred slowly in this beaker using a Rayneri mixer.
The Rayneri mixer is a laboratory stirrer equipped with a paddle or turbine which makes possible stirring at variable speed while constituents are being incorporated.
The polymers, the fillers and then the surface-active agents are successively introduced, still with slow stirring. Between each introduction, care is taken to ensure good dissolution of the compound and homogenization of the medium.
When all the compounds are dissolved, it is necessary to adjust the weight of water in order to compensate for the evaporation which has taken place. The preserving system is then added.
The aqueous phase is then placed on a water bath until a temperature of 85-90°C is reached.

*iii. Emulsification*

When the two phases are at the desired temperature, the aqueous phase is added very slowly to the fatty phase while gradually increasing the stirring; phase inversion is then visible by a change in viscosity. The stirring is adjusted to the maximum for 10 minutes (approximately 3500 rev/min) but, for certain formulations, there is a risk of overflowing if the stirring is too vigorous. In order for the emulsion to be homogeneous, it is sometimes necessary to use a flexible spatula to scrape the edges and to bring the product to the centre in order for it all to be entrained by the paddle.

*iv. Temperature reduction*

After the emulsification, the heating pan is placed on the Rayneri mixer equipped then with a butterfly paddle; the latter makes possible kneading and homogenization during the temperature reduction, at low shear. The stirring speed is low in order not to incorporate air bubbles.
The temperature is gradually reduced using the oil bath: by stationary phases of 10°C, homogenizing thoroughly at each stationary phase, especially at about 70°C (critical temperature where the mascara begins to significantly set solid). It is sometimes necessary to mix using the flexible spatula in order to avoid the formation of a block around the paddle which would then no longer be kneaded and would be nonhomogeneous. However, there is a risk of incorporation of air bubbles by this method; the formulator must then find the correct compromise between homogeneity and air bubbles.

*v. End of formulation*

The mascara thus obtained is transferred into a closed jar in order to prevent it from drying on contact with the air; it is then necessary to wait 24 hours in order to confirm that the formulation is homogeneous and that the pigments are correctly dispersed.

2/ Protocols and Results

In this example, the waxes and the emulsifying system are present at a respective total content such that the ratio by weight of the waxes to the emulsifying system is equal to 0.55, for a solids content of 39.10% and rheology G* of 14 000 Pa at T0.
The composition, despite a high solids content and in particular a high content of film-forming polymer, thus exhibits a smooth appearance, has a good texture and makes possible, on application, a volume, lengthening and

curving effect, with an even structure of the layers of mascara deposited and a good playtime.

Throughout the patent application, every weight of compound is expressed by weight of dry matter of the compound in question, unless deliberately mentioned.

Throughout the patent application, the wording "comprising a" means "comprising at least one", unless otherwise specified.

## Claims

1. Cosmetic composition for coating keratinous fibres of the emulsion type, comprising:

   - an aqueous phase,
   - at least one wax,
   - an emulsifying system comprising i) at least one non-ionic surfactant exhibiting, at 25°C, an HLB balance within the Griffin meaning of greater than or equal to 8, ii) at least one anionic surfactant comprising at least one cationic counterion, the said anionic surfactant(s) is (are) chosen from $C_{12}$-$C_{22}$, fatty acids, iii) at least one co-surfactant, the co-surfactant(s) is (are) chosen from fatty alcohols comprising from 10 to 26 carbon atoms, iv) a non-ionic surfactant exhibiting, at 25°C, an HLB balance within the Griffin meaning of less than 8,
   - at least one film-forming polymer present in a solids content of greater than or equal to 5% by weight, with respect to the total weight of the composition,
   - at least one polymeric organic lipophilic gelling agent chosen from polycondensates of hydrocarbon polyamide type,

   in which the wax(es) and the emulsifying system are present at a respective total content such that the ratio by weight of the wax(es) to the emulsifying system is less than or equal to 1.

2. Composition according to Claim 1, in which the said anionic surfactant(s) is (are) chosen from $C_{14}$-$C_{18}$ fatty acids, and preferably comprises stearic acid.

3. Composition according to Claim 1 or 2, in which the said anionic surfactant(s) is (are) present at a total content of greater than or equal to 1% by weight, with respect to the total weight of the composition, preferably ranging from 1.5% to 8% by weight and better still from 2% to 5% by weight, with respect to the total weight of the composition.

4. Composition according to any one of Claims 1 to 3, in which the said cationic counterion(s) is (are) chosen from a cation of inorganic origin, in particular chosen from alkali metal and alkaline earth metal cations, or of organic origin, preferably of organic origin.

5. Composition according to any one of Claims 1 to 4 in which the said cationic counterion(s) is (are) chosen from ammonium, and its amine and aminoalcohol derivatives, or magnesium, preferably from ammonium, and its amine and aminoalcohol derivatives.

6. Composition according to any one of Claims 1 to 5, in which the said cationic counterion(s) comprise(s) a primary (poly)hydroxyalkylamine, preferably aminomethyl propanediol.

7. Composition according to any one of Claims 1 to 6, in which the total content of cationic counterion(s) is greater than or equal to 0.01% by weight, preferably inclusively between 0.1% and 4% by weight and better still between 0.5% and 2% by weight, with respect to the total weight of the composition.

8. Composition according to any one of the preceding claims, in which the non-ionic surfactant(s) exhibiting, at 25°C, an HLB balance within the Griffin meaning of greater than or equal to 8 are chosen from:

   - oxyethylenated and/or oxypropylenated glycerol ethers which can comprise from 1 to 150 oxyethylene and/or oxypropylene units;
   - oxyalkylenated, in particular oxyethylenated and/or oxypropylenated, alcohols which can comprise from 1 to 150 oxyethylene and/or oxypropylene units, preferably from 20 to 100 oxyethylene units;
   - esters of fatty acid, in particular $C_8$-$C_{24}$ and preferably $C_{16}$-$C_{22}$ fatty acid, and of polyethylene glycol (or PEG);
   - esters of fatty acid, in particular $C_8$-$C_{24}$ and preferably $C_{16}$-$C_{22}$ fatty acid, and of glycerol ethers which are oxyethylenated and/or oxypropylenated, preferably oxyethylenated;

- esters of fatty acid, in particular $C_8$-$C_{24}$ and preferably $C_{16}$-$C_{22}$ fatty acid, and of sorbitol ethers which are oxyethylenated and/or oxypropylenated, preferably oxyethylenated;
- dimethicone copolyol;
- dimethicone copolyol benzoate;
- copolymers of propylene oxide and of ethylene oxide, also known as EO/PO polycondensates;
- and their mixtures.

**9.** Composition according to any one of the preceding claims, in which the non-ionic surfactant(s) exhibiting, at 25°C, an HLB balance within the Griffin meaning of greater than or equal to 8 is (are) present at a total content of greater than or equal to 1% by weight, with respect to the total weight of the composition, preferably ranging from 1.5% to 8% by weight and better still from 2% to 5% by weight, with respect to the total weight of the composition.

**10.** Composition according to any one of the preceding claims, in which the the non-ionic surfactant(s) exhibiting, at 25°C, an HLB balance within the Griffin meaning of less than 8 are chosen from:

- saccharide esters and ethers;
- esters of fatty acids, in particular $C_8$-$C_{24}$ and preferably $C_{16}$-$C_{22}$ fatty acids, and of polyol, in particular of glycerol or sorbitol, preferably of glycerol;
- oxyalkylenated, in particular oxyethylenated and/or oxypropylenated, alcohols;
- a cyclomethicone/dimethicone copolyol mixture;
- and their mixtures.

**11.** Composition according to any one of the preceding claims, in which the non-ionic surfactant(s) exhibiting, at 25°C, an HLB balance within the Griffin meaning of less than 8 is (are) present at a total content of greater than or equal to 1% by weight, with respect to the total weight of the composition, preferably ranging from 1.5% to 8% by weight and better still from 2% to 5% by weight, with respect to the total weight of the composition.

**12.** Composition according to any one of the preceding claims, in which the co-surfactant(s) is (are) chosen from fatty alcohols comprising from 12 to 24 carbon atoms and even better still from 14 to 22 carbon atoms.

**13.** Composition according to any one of the preceding claims, in which the said wax(es) is (are) present at a total content of less than or equal to 12% by weight, with respect to the total weight of the said composition, preferably inclusively between 5% and 8% by weight, with respect to the total weight of the composition.

**14.** Composition according to any one of the preceding claims, the film-forming polymer(s) comprising at least one aqueous dispersion of film-forming polymer.

**15.** Composition according to any one of the preceding claims, in which the film-forming polymer(s) comprise(s) at least two aqueous dispersions of film-forming polymer, at least one being an aqueous dispersion of acrylic polymer and at least the other being an aqueous dispersion of polyurethane, and their derivatives.

**16.** Composition according to any one of the preceding claims, in which the organic lipophilic gelling agent(s) is (are) present at a content ranging from 0.5% to 6% by weight, on a dry basis, with respect to the total weight of the composition.

**17.** Composition according to the preceding claim, comprising at least one inorganic filler advantageously chosen from talc, mica, synthetic fluorphlogopite, a clay, such as smectite, and their mixtures.

**18.** Method for coating keratinous fibres, in particular for making up the eyelashes, comprising a stage of application of a cosmetic composition for coating keratinous fibres according to any one of Claims 1 to 17.

**Patentansprüche**

**1.** Kosmetische Zusammensetzung zum Beschichten von Keratinfasern vom Emulsionstyp, umfassend:

- eine wässrige Phase,
- mindestens ein Wachs,

- ein Emulgiersystem, umfassend i) mindestens ein nichtionisches Tensid, das bei 25 °C eine HLB-Balance innerhalb der Griffin-Bedeutung größer oder gleich 8 aufweist, ii) mindestens ein anionisches Tensid mit mindestens einem kationischen Gegenion, wobei das anionische Tensid bzw. die anionischen Tenside aus $C_{12}$-$C_{22}$-Fettsäuren ausgewählt ist bzw. sind, iii) mindestens ein Cotensid wobei das Cotensid bzw. die Cotenside aus Fettalkoholen mit 10 bis 26 Kohlenstoffatomen ausgewählt ist bzw. sind, iv) ein nichtionisches Tensid, das bei 25 °C eine HLB-Balance innerhalb der Griffin-Bedeutung von weniger als 8 aufweist,
- mindestens ein filmbildendes Polymer, das in einem Feststoffgehalt größer oder gleich 5 Gew.-%, bezogen auf das Gesamtgewicht der Zusammensetzung, vorliegt,
- mindestens ein polymeres organisches lipophiles Gelierungsmittel, das aus Polykondensaten vom Kohlenwasserstoffpolyamidtyp ausgewählt ist,

wobei das Wachs bzw. die Wachse und das Emulgiersystem in einem solchen jeweiligen Gesamtgehalt vorliegen, dass das Gewichtsverhältnis des Wachses bzw. der Wachse zu dem Emulgiersystem kleiner oder gleich 1 ist.

**2.** Zusammensetzung nach Anspruch 1, wobei das anionische Tensid bzw. die anionischen Tenside aus $C_{14}$-$C_{18}$-Fettsäuren ausgewählt ist bzw. sind und vorzugsweise Stearinsäure umfasst bzw. umfassen.

**3.** Zusammensetzung nach Anspruch 1 oder 2, wobei das anionische Tensid bzw. die anionischen Tenside in einem Gesamtgehalt größer oder gleich 1 Gew.-%, bezogen auf das Gesamtgewicht der Zusammensetzung, vorzugsweise im Bereich von 1,5 bis 8 Gew.-% und noch besser von 2 bis 5 Gew.-%, bezogen auf das Gesamtgewicht der Zusammensetzung, vorliegt bzw. vorliegen.

**4.** Zusammensetzung nach einem der Ansprüche 1 bis 3, wobei das kationische Gegenion bzw. die kationischen Gegenionen aus einem Kation anorganischen Ursprungs, das insbesondere aus Alkalimetall- und Erdalkalimetallkationen ausgewählt ist, oder organischen Ursprungs, vorzugsweise organischen Ursprungs, ausgewählt ist bzw. sind.

**5.** Zusammensetzung nach einem der Ansprüche 1 bis 4, wobei das kationische Gegenion bzw. die kationischen Gegenionen aus Ammonium und dessen Amin- und Aminoalkohol-Derivaten oder Magnesium, vorzugsweise aus Ammonium und dessen Amin- und Aminoalkohol-Derivaten, ausgewählt ist.

**6.** Zusammensetzung nach einem der Ansprüche 1 bis 5, wobei das kationische Gegenion bzw. die kationischen Gegenionen ein primäres (Poly)-hydroxyalkylamin, vorzugsweise Aminomethylpropandiol, umfasst bzw. umfassen.

**7.** Zusammensetzung nach einem der Ansprüche 1 bis 6, wobei der Gesamtgehalt an kationischem Gegenion bzw. kationischen Gegenionen größer oder gleich 0,01 Gew.-% ist und vorzugsweise inklusive zwischen 0,1 und 4 Gew.-% und noch besser zwischen 0,5 und 2 Gew.-%, bezogen auf das Gesamtgewicht der Zusammensetzung, liegt.

**8.** Zusammensetzung nach einem der vorhergehenden Ansprüche, wobei das nichtionische Tensid bzw. die nichtionischen Tenside, das bzw. die bei 25 °C eine HLB-Balance innerhalb der Griffin-Bedeutung größer oder gleich 8 aufweist bzw. aufweisen, aus

- oxyethylenierten und/oder oxypropylenierten Glycerinethern, die 1 bis 150 Oxyethylen- und/oder Oxypropylen-Einheiten umfassen können;
- oxyalkylenierten, insbesondere oxyethylenierten und/oder oxypropylenierten, Alkoholen, die 1 bis 150 Oxyethylen- und/oder Oxypropylen-Einheiten, vorzugsweise 20 bis 100 Oxyethylen-Einheiten, umfassen können;
- Estern von Fettsäuren, insbesondere $C_8$-$C_{24}$- und vorzugsweise $C_{16}$-$C_{22}$-Fettsäure, und von Polyethylenglykol (oder PEG);
- Estern von Fettsäuren, insbesondere $C_8$-$C_{24}$- und vorzugsweise $C_{16}$-$C_{22}$-Fettsäure, und von Glycerinethern, die oxyethyleniert und/oder oxypropyleniert, vorzugsweise oxyethyleniert, sind;
- Estern von Fettsäuren, insbesondere $C_8$-$C_{24}$- und vorzugsweise $C_{16}$-$C_{22}$-Fettsäure, und von Sorbitolethern, die oxyethyleniert und/oder oxypropyleniert, vorzugsweise oxyethyleniert, sind;
- Dimethiconcopolyol;
- Dimethiconcopolyolbenzoat;
- Copolymeren von Propylenoxid und von Ethylenoxid, die auch als EO/PO-Polykondensate bekannt sind;
- und Mischungen davon

ausgewählt ist bzw. sind.

**9.** Zusammensetzung nach einem der vorhergehenden Ansprüche, wobei das nichtionische Tensid bzw. die nichtionischen Tenside, das bzw. die bei 25 °C eine HLB-Balance innerhalb der Griffin-Bedeutung größer oder gleich 8 aufweist bzw. aufweisen, in einem Gesamtgehalt größer oder gleich 1 Gew.-%, bezogen auf das Gesamtgewicht der Zusammensetzung, vorzugsweise im Bereich von 1,5 bis 8 Gew.-% und noch besser von 2 bis 5 Gew.-%, bezogen auf das Gesamtgewicht der Zusammensetzung, vorliegt bzw. vorliegen.

**10.** Zusammensetzung nach einem der vorhergehenden Ansprüche, wobei das nichtionische Tensid bzw. die nichtionischen Tenside, das bzw. die bei 25 °C eine HLB-Balance innerhalb der Griffin-Bedeutung von weniger 8 aufweist bzw. aufweisen, aus

- Saccharidestern und -ethern,
- Estern von Fettsäuren, insbesondere $C_8$-$C_{24}$- und vorzugsweise $C_{16}$-$C_{22}$-Fettsäure, und von Polyol, insbesondere von Glycerin oder Sorbitol, vorzugsweise von Glycerin;
- oxyalkylenierten, insbesondere oxyethylenierten und/oder oxypropylenierten, Alkoholen;
- einer Cyclomethicon/Dimethiconcopolyol-Mischung;
- und Mischungen davon

ausgewählt ist bzw. sind.

**11.** Zusammensetzung nach einem der vorhergehenden Ansprüche, wobei das nichtionische Tensid bzw. die nichtionischen Tenside, das bzw. die bei 25 °C eine HLB-Balance innerhalb der Griffin-Bedeutung von weniger als 8 aufweist bzw. aufweisen, in einem Gesamtgehalt größer oder gleich 1 Gew.-%, bezogen auf das Gesamtgewicht der Zusammensetzung, vorzugsweise im Bereich von 1,5 bis 8 Gew.-% und noch besser von 2 bis 5 Gew.-%, bezogen auf das Gesamtgewicht der Zusammensetzung, vorliegt bzw. vorliegen.

**12.** Zusammensetzung nach einem der vorhergehenden Ansprüche, wobei das Cotensid bzw. die Cotenside aus Fettalkoholen mit 12 bis 24 Kohlenstoffatomen und noch besser 14 bis 22 Kohlenstoffatomen ausgewählt ist bzw. sind.

**13.** Zusammensetzung nach einem der vorhergehenden Ansprüche, wobei das Wachs bzw. die Wachse in einem Gesamtgehalt kleiner oder gleich 12 Gew.-%, bezogen auf das Gesamtgewicht der Zusammensetzung, vorzugsweise inklusive zwischen 5 und 8 Gew.-%, bezogen auf das Gesamtgewicht der Zusammensetzung, vorliegt bzw. vorliegen.

**14.** Zusammensetzung nach einem der vorhergehenden Ansprüche, wobei das filmbildende Polymer bzw. die filmbildenden Polymere mindestens eine wässrige Dispersion von filmbildendem Polymer umfasst bzw. umfassen.

**15.** Zusammensetzung nach einem der vorhergehenden Ansprüche, wobei das filmbildende Polymer bzw. die filmbildenden Polymere mindestens zwei wässrige Dispersionen von filmbildendem Polymer umfasst bzw. umfassen, wobei mindestens eine eine wässrige Dispersion von Acrylpolymer und mindestens die andere eine wässrige Dispersion von Polyurethan und Derivaten davon ist.

**16.** Zusammensetzung nach einem der vorhergehenden Ansprüche, wobei das organische lipophile Geliermittel bzw. die organischen lipophilen Geliermittel in einem Gehalt im Bereich von 0,5 bis 6 Gew.-% auf Trockenbasis, bezogen auf das Gesamtgewicht der Zusammensetzung, vorliegt bzw. vorliegen.

**17.** Zusammensetzung nach dem vorhergehenden Anspruch, umfassend mindestens einen anorganischen Füllstoff, der vorteilhafterweise aus Talk, Glimmer, synthetischem Fluorphlogopit, einem Ton, wie Smektit, und Mischungen davon ausgewählt ist.

**18.** Verfahren zum Beschichten von Keratinfasern, insbesondere zum Schminken der Wimpern, umfassend eine Stufe des Aufbringens einer kosmetischen Zusammensetzung zum Beschichten von Keratinfasern nach einem der Ansprüche 1 bis 17.

## Revendications

**1.** Composition cosmétique de revêtement des fibres kératiniques, de type émulsion, comprenant :

- une phase aqueuse,
- au moins une cire,
- un système émulsifiant, comprenant i) au moins un agent tensioactif non ionique présentant à 25°C une balance HLB au sens de Griffin supérieure ou égale à 8, ii) au moins un agent tensioactif anionique comprenant au moins un contre-ion cationique, ledit ou lesdits agents tensioactifs anioniques étant choisis parmi les acides gras en $C_{12}$-$C_{22}$, iii) au moins un co-agent tensioactif, le ou les co-agents tensioactifs étant choisis parmi les alcools gras comprenant de 10 à 26 atomes de carbone, iv) un agent tensioactif non ionique présentant à 25°C une balance HLB au sens de Griffin inférieure à 8,
- au moins un polymère filmogène présent selon une teneur en matière sèche supérieure ou égale à 5% en poids, par rapport au poids total de la composition,
- au moins un agent gélifiant lipophile organique polymère, choisi parmi les polycondensats de type polyamide hydrocarboné,

dans laquelle la ou les cires et le système émulsifiant sont présents selon une teneur totale respective telle que le rapport pondéral de la ou des cires au système émulsifiant est inférieur ou égal à 1.

**2.** Composition selon la revendication 1, dans laquelle ledit ou lesdits agents tensioactifs anioniques sont choisis parmi les acides gras en $C_{14}$-$C_{18}$, et comprennent de préférence l'acide stéarique.

**3.** Composition selon la revendication 1 ou 2, dans laquelle ledit ou lesdits agents tensioactifs anioniques sont présents selon une teneur totale supérieure ou égale à 1% en poids, par rapport au poids total de la composition, préférablement allant de 1,5% à 8% en poids et mieux encore de 2% à 5% en poids, par rapport au poids total de la composition.

**4.** Composition selon l'une quelconque des revendications 1 à 3, dans laquelle ledit ou lesdits contre-ions cationiques sont choisis parmi un cation d'origine inorganique, en particulier choisi parmi les cations de métaux alcalins ou de métaux alcalino-terreux, ou d'origine organique, préférablement d'origine organique.

**5.** Composition selon l'une quelconque des revendications 1 à 4, dans laquelle ledit ou lesdits contre-ions cationiques sont choisis parmi l'ammonium, et ses dérivés d'amine et d'aminoalcool, ou le magnésium, préférablement parmi l'ammonium, et ses dérivés d'amine et d'aminoalcool.

**6.** Composition selon l'une quelconque des revendications 1 à 5, dans laquelle ledit ou lesdits contre-ions cationiques comprennent une (poly)hydroxy-alkylamine primaire, préférablement l'aminométhyl propanediol.

**7.** Composition selon l'une quelconque des revendications 1 à 6, dans laquelle la teneur totale en contre-ion(s) cationique(s) est supérieure ou égale à 0,01% en poids, de préférence comprise inclusivement entre 0,1% et 4% en poids, et mieux encore entre 0,5% et 2% en poids, par rapport au poids total de la composition.

**8.** Composition selon l'une quelconque des revendications précédentes, dans laquelle le ou les agents tensioactifs non ioniques présentant à 25°C une balance HLB au sens de Griffin supérieure ou égale à 8, sont choisis parmi

- les éthers de glycérol oxyéthylénés et/ou oxypropylénés, pouvant comporter de 1 à 150 motifs oxyéthylène et/ou oxypropylène ;
- les alcools oxyalkylénés, en particulier oxyéthylénés et/ou oxypropylénés, pouvant comporter de 1 à 150 motifs oxyéthylène et/ou oxypropylène, de préférence de 20 à 100 motifs oxyéthylène ;
- les esters d'acides gras, en particulier d'acides gras en $C_8$-$C_{24}$, et de préférence en $C_{16}$-$C_{22}$, et de polyéthylène glycol (ou PEG) ;
- les esters d'acides gras, en particulier d'acides gras en $C_8$-$C_{24}$, et de préférence en $C_{16}$-$C_{22}$, et d'éthers de glycérol qui sont oxyéthylénés et/ou oxypropylénés, de préférence oxyéthylénés ;
- les esters d'acides gras, en particulier d'acides gras en $C_8$-$C_{24}$, et de préférence en $C_{16}$-$C_{22}$, et d'éthers de sorbitol qui sont oxyéthylénés et/ou oxypropylénés, de préférence oxyéthylénés ;
- le diméthicone copolyol ;
- le diméthicone copolyol benzoate ;
- les copolymères d'oxyde de propylène et d'oxyde d'éthylène, également appelés polycondensats d'OE/OP,
- et leurs mélanges.

**9.** Composition selon l'une quelconque des revendications précédentes, dans laquelle le ou les agents tensioactifs

non ioniques présentant à 25°C une balance HLB au sens de Griffin supérieure ou égale à 8 sont présents selon une teneur totale supérieure ou égale à 1% en poids, par rapport au poids total de la composition, de préférence allant de 1,5% à 8% en poids, et mieux encore de 2% à 5% en poids, par rapport au poids total de la composition.

**10.** Composition selon l'une quelconque des revendications précédentes, dans laquelle le ou les agents tensioactifs non ioniques présentant à 25°C une balance HLB au sens de Griffin inférieure à 8, sont choisis parmi

- les esters et éthers de saccharides ;
- les esters d'acides gras, en particulier d'acides gras en $C_8$-$C_{24}$, et de préférence en $C_{16}$-$C_{22}$, et de polyol, en particulier de glycérol ou de sorbitol, de préférence de glycérol ;
- les alcools oxyalkylénés, en particulier oxyéthylénés et/ou oxypropylénés ;
- un mélange de cyclométhicone/diméthicone copolyol ;
- et leurs mélanges.

**11.** Composition selon l'une quelconque des revendications précédentes, dans laquelle le ou les agents tensioactifs non ioniques présentant à 25°C une balance HLB au sens de Griffin inférieure à 8 sont présents selon une teneur totale supérieure ou égale à 1% en poids, par rapport au poids total de la composition, de préférence allant de 1,5% à 8% en poids, et mieux encore de 2% à 5% en poids, par rapport au poids total de la composition.

**12.** Composition selon l'une quelconque des revendications précédentes, dans laquelle le ou les co-agents tensioactifs sont choisis parmi les alcools gras comprenant de 12 à 24 atomes de carbone et mieux encore de 14 à 22 atomes de carbone.

**13.** Composition selon l'une quelconque des revendications précédentes, dans laquelle ladite ou lesdites cires sont présentes selon une teneur totale inférieure ou égale à 12% en poids, par rapport au poids total de ladite composition, de préférence comprise inclusivement entre 5% et 8% en poids, par rapport au poids total de la composition.

**14.** Composition selon l'une quelconque des revendications précédentes, le ou les polymères filmogènes comprenant au moins une dispersion aqueuse de polymère filmogène.

**15.** Composition selon l'une quelconque des revendications précédentes, dans laquelle le ou les polymères filmogènes comprennent au moins deux dispersions aqueuses de polymère filmogène, au moins l'une étant une dispersion aqueuse de polymère acrylique et au moins l'autre étant une dispersion aqueuse de polyuréthane, et leurs dérivés.

**16.** Composition selon l'une quelconque des revendications précédentes, dans laquelle le ou les agents gélifiants lipophiles organiques sont présents selon une teneur allant de 0,5% à 6% en poids, sur une base de matière sèche, par rapport au poids total de la composition.

**17.** Composition selon la revendication précédente, comprenant au moins une charge inorganique, choisie de manière avantageuse parmi le talc, le mica, la fluorphlogopite synthétique, une argile telle que la smectite, et leurs mélanges.

**18.** Méthode de revêtement des fibres kératiniques, en particulier de maquillage des cils, comprenant une étape d'application d'une composition cosmétique de revêtement des fibres kératiniques selon l'une quelconque des revendications 1 à 17.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010002602 A2
- FR 2792190 A
- WO 2007068371 A
- WO 2008155059 A
- US 5188899 A
- US 3412054 A


### Non-patent literature cited in the description


- Microemulsions Theory and Practice. Academic Press, 1977, 21-32
- **GRIFFIN.** *J. Soc. Cosm. Chem.,* 1954, vol. 5, 249-256
- Kirk-Othmer Encyclopedia of Chemical Technology. Wiley, 1979, vol. 22, 333-432